(19) Europäisches Patentamt
European Patent Office
Office européen des brevets

(11) EP 2 191 830 A1

(12) **EUROPEAN PATENT APPLICATION**
published in accordance with Art. 153(4) EPC

(43) Date of publication:
**02.06.2010 Bulletin 2010/22**

(21) Application number: **08831578.3**

(22) Date of filing: **18.09.2008**

(51) Int Cl.:
***A61K 31/44** (2006.01)*
***A61K 47/16** (2006.01)*
***A61K 47/22** (2006.01)*
***A61K 47/38** (2006.01)*
***A61P 25/02** (2006.01)*
***A61P 25/16** (2006.01)*
***A61P 25/22** (2006.01)*
***A61P 25/24** (2006.01)*
***A61P 25/28** (2006.01)*
***A61P 43/00** (2006.01)*
***C07D 213/76** (2006.01)*

(86) International application number:
**PCT/JP2008/066818**

(87) International publication number:
**WO 2009/038112 (26.03.2009 Gazette 2009/13)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **21.09.2007 JP 2007244949**

(71) Applicant: **Shionogi&Co., Ltd.**
**Osaka-shi, Osaka 5410045 (JP)**

(72) Inventors:
- **TAKADA, Masatoshi**
**Amagasaki-shi**
**Hyogo 660-0813 (JP)**
- **MURAKAMI, Yuki**
**Amagasaki-shi**
**Hyogo 660-0813 (JP)**
- **ICHIO, Shunji**
**Amagasaki-shi**
**Hyogo 660-0813 (JP)**
- **FUJII, Minako**
**Amagasaki-shi**
**Hyogo 660-0813 (JP)**
- **TAKAKURA, Asako**
**Amagasaki-shi**
**Hyogo 660-0813 (JP)**
- **FUNAKI, Takeshi**
**Amagasaki-shi**
**Hyogo 660-0813 (JP)**

(74) Representative: **Vossius & Partner**
**Siebertstraße 4**
**81675 München (DE)**

# (54) SOLID PREPARATION COMPRISING NPYY5 RECEPTOR ANTAGONIST

(57) A preparation which can improve solubility of a NPYY5 receptor antagonist in water, even when the NPYY5 receptor antagonist is contained in the preparation at a high content is provided. A solid preparation containing a NPYY5 receptor antagonist, an amorphous stabilizer, and optionally an amorphousization inducing agent. Particularly, when the amorphous stabilizer is hydroxypropylmethylcellulose phthalate and/or hydroxypropylmethylcellulose acetate succinate, and the amorphousization inducing agent is urea and/or saccharine sodium at an addition amount of less than 8% by weight, dissolution out property of a water-hardly soluble NPYY5 receptor antagonist could be improved.



Printed by Jouve, 75001 PARIS (FR)

## Description

[Technical field]

The present invention relates to a preparation for improving solubility of a NPYY5 receptor antagonist in water. More particularly, the present invention relates to a solid preparation of a NPYY5 receptor antagonist containing an amorphous stabilizer, or an amorphous stabilizer and an amorphousization inducing agent.

[Background art]

Neuropeptide Y (hereinafter, referred to as NPY) is a peptide consisting of 36 amino acid residues, and was separated from a pig brain in 1982. NPY is widely distributed in a central nervous system and a peripheral tissue of a human and an animal.
In the previous reports, it has been found out that NPY has ingestion promotion activity, anti-spasm activity, learning promotion activity, anti-anxiety activity, anti-stress activity and the like in a central nervous system and, further, there is a possibility that NPY is deeply involved in a central nervous system disease such as depression, Alzheimer-type dementia, Parkinson's disease and the like. In addition, in a peripheral tissue, since NPY causes constriction of a smooth muscle of a blood vessel or the like, and a cardiac muscle, it is thought that NPY is also involved in a circulatory system disease. Further, it is known that NPY is involved in a metabolic disease such as obesity, diabetes, hormone abnormality or the like (Trends in Pharmacological Sciences, Vol.15, 153 (1994)). Therefore, there is a possibility that the NPY receptor antagonist serves as a drug for preventing or treating various diseases with which a NPY receptor is involved, such as those described above.
As the NPY receptor, subtypes ofY1, Y2, Y3, Y4, Y5 and Y6 have been currently discovered (Trends in Pharmacological Sciences, Vol.18, 372 (1997)). AY5 receptor is involved at least in ingestion function, and it has been suggested that an antagonist thereof serves as an anti-obesity drug (Peptides, Vol.18, 445 (1997)).
As this NPYY5 receptor antagonist, compounds described in International Publication Pamphlet WO 01/37826 are exemplified and, particularly, trans-N-(5-trifluoromethylpyridin-2-yl)-4-(tertiary butylsulfonylamino)cyclohexanecarboxamide exhibits high anti-obesity effect. The present drug is orally administered and, according to study by the present inventors, it has been revealed that since the drug has low solubility in water, and it is not sufficiently dissolved in a digestive tract, an absorption amount is reduced. Since by such the drug, as a dose is increased, an absorption fraction of the drug is reduced, and an absorption fraction easily varies due to digestion working at ingestion (mechanical stimulation due to constriction motion of a digestive tract, increase in a secretion amount of a digestive fluid, prolongation of a digestive tract retention time) as compared with at hunger, an expected therapeutic effect is not obtained, or an occasional harmful effect is caused in some cases. For this reason, particularly, increase in a rate of dissolution of a drug from a solid preparation is important for developing an oral preparation.
As one of means for improving solubility of a poorly water-soluble drug in a solid preparation in water, a solid dispersion in which a drug molecule is uniformly dispersed in a base of the solid state, and a drug molecule is in the state where it does not form a crystal (amorphous) is used. As such a solid dispersion, a preparation containing a drug, an amorphous stabilizer and an amorphousization inducing agent is being studied. For example, Patent Document 1 discloses a solid dispersion obtained by heating or mechanochemically treating a preparation containing nicardipine hydrochloride, 15% by weight of urea as an amorphousization inducing agent, and hydroxypropylmethylcellulose as an amorphousization stabilizer. In addition, Patent Document 2 discloses a solid dispersion obtained by heating or mechanochemically treating a preparation containing efonidipine hydrochloride, 11% by weight of urea as an amorphousization inducing agent, and hydroxypropylmethylcellulose acetate succinate as an amorphous stabilizer. Further, Non-patent Document 1 discloses a solid dispersion containing nifedipine, polyvinylpyrrolidone and 7% by weight of urea.
However, solid dispersions of Patent Documents 1 and 2 are such that a production process is a method of giving a burden excessive for a drug, such as high temperature heating and mechanochemical treatment and, therefore, there is a possibility of degradation of a drug. In addition, Patent Document 3 describes increase in solubility of efonidipine hydrochloride in water, Patent Document 4 describes increase in solubility cyclosporine A in water, Patent Document 5 describes increase in solubility of bicalutamide in water, Patent Document 6 describes increase in solubility of amifostine in water, and Non-patent Document 1 describes increase in solubility of nifedipine in water, but a kind of an optimal amorphous stabilizer and amorphousization inducing agent, and an optimal blending amount are not necessarily the same, depending on a drug. In addition, Patent Document 7 describes a solid dispersion containing a poorly water-soluble drug, but a preparation of a NPYY5 receptor antagonist blended in the present preparation is not specifically described.
[Patent Document 1] International Publication WO 97/06781

[Patent Document 2] Japanese Patent Application Laid-Open (JP-A) No.9-309834
[Patent Document 3] JP-A No.2-49728
[Patent Document 4] JP-A No.2004-528358
[Patent Document 5] JP-A No.2004-143185
[Patent Document 6] JP-A No.2002-529519
[Patent Document 7] International Publication WO 2007/108463
[Non-Patent Document 1] 2005 Report of Important Study of Drug Design etc. Human Science Study, Third Field, Objective No. KH31024 (publication date: July 31, 2006)

[Disclosure of the invention]

[Problems to be solved by the invention]

Therefore, development of a novel solid preparation having improved solubility of a NPYY5 receptor antagonist has been demanded.

[Means to solve the problems]

Then, the present inventors intensively studies, and found out that solubility of a drug can be improved by containing an amorphous stabilizer and, optionally, an amorphousization inducing agent in a solid preparation of a NPYY5 receptor antagonist.
Preferably, by containing hydroxypropylmethylcellulose phthalate (hereinafter, also referred to as HPMCP) and/or hydroxypropylmethylcellulose acetate succinate (hereinafter, also referred to as HPMCAS) as an amorphous stabilizer, and urea and/or saccharine sodium as an amorphousization inducing agent, solubility of the NPYY5 receptor antagonist, particularly, trans-N-(5-trifluoromethylpyridin-2-yl)-4-(tertiary butylsulfonylamino)cyclohexanecarboxamide (hereinafter, also referred to as S-2367) can be improved.
That is, the present invention relates to:

(1) a solid preparation comprising a compound represented by the formula (I):

[Chemical formula 1]

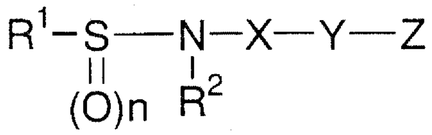

(I)

[wherein,
$R^1$ is lower alkyl optionally having a substituent, cycloalkyl optionally having a substituent, or aryl optionally having a substituent,
$R^2$ is hydrogen or lower alkyl,
$R^1$ and $R^2$ may be taken together to form lower alkylene,
n is 1 or 2,
X is lower alkylene optionally having a substituent, lower alkenylene optionally having a substituent, -CO-lower alkylene optionally having a substituent, -CO-lower alkenylene optionally having a substituent, or

[Chemical formula 2]

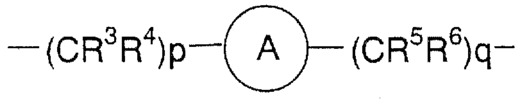

(wherein $R^3$, $R^4$, $R^5$ and $R^6$ are each independently hydrogen or lower alkyl, wherein

[Chemical formula 3]

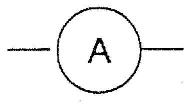

is cycloalkylene optionally having a substituent, cycloalkenylene optionally having a substituent, bicycloalkylene optionally having a substituent, arylene optionally having a substituent, or heterocyclic diyl optionally having a substituent, and p and q are each independently 0 or 1)
$-NR^2-X-$ is

[Chemical formula 4]

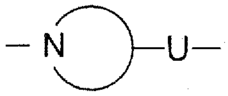

(wherein

[Chemical formula 5]

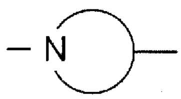

is piperidinediyl, piperazinediyl, pyridinediyl, pyrazinediyl, pyrrolidinediyl or pyrrolediyl, and U is a single bond, lower alkylene or lower alkenylene),
Y is $OCONR^7$, $CONR^7$, $CSNR^7$, $NR^7CO$ or $NR^7CS$,
$R^7$ is hydrogen or lower alkyl,
Z is lower alkyl optionally having a substituent, lower alkenyl optionally having a substituent, amino optionally having a substituent, lower alkoxy optionally having a substituent, a hydrocarbon cyclic group optionally having a substituent, or a heterocyclic group optionally having a substituent]
or a prodrug thereof, or a pharmaceutically acceptable salt thereof, or a solvate thereof, and an amorphous stabilizer,
(2) the solid preparation according to (1), wherein the amorphous stabilizer is one or more selected from the group consisting of polyvinylpyrrolidone, celluloses, crosslinked polyvinylpyrrolidone, polyvinyl alcohol, polyvinyl acetate, vinyl alcohol/vinyl acetate copolymer, ethylene/vinyl acetate copolymer, polyethylene oxide derivative, sodium polystyrenesulfonate, gelatin, starch, dextran, agar, sodium alginate, pectin, pullulan, xanthan gum, acacia, chondroitin sulfate or a sodium salt thereof, hyaluronic acid, chitin, chitosan, $\alpha$, $\beta$ or $\gamma$-cyclodextrin, alginic acid derivative, acryl resins, polyvinylacetal diethylaminoacetate, silicon dioxide, carrageenan and aluminum hydroxide,
(3) the solid preparation according to (1) or (2), wherein the amorphous stabilizer is one or more selected from the group consisting of polyvinylpyrrolidone, celluloses, polyvinyl alcohol, polyvinyl acetate, gelatin, agar, sodium alginate, pectin, pullulan, xanthan gum, acacia, chondroitin sulfate, hyaluronic acid and carrageenan,
(4) the solid preparation according to any one of (1) to (3), wherein the amorphous stabilizer is one or more celluloses selected from the group consisting of hydroxypropylcellulose, hydroxypropylmethylcellulose, hydroxypropylmethylcellulose phthalate, hydroxypropylmethylcellulose acetate succinate and carboxymethylcellulose sodium,
(5) the solid preparation according to (4), wherein the amorphous stabilizer is hydroxypropylmethylcellulose phthalate

and/or hydroxypropylmethylcellulose acetate succinate,

(6) the solid preparation according to any one of (1) to (5), which further contains an amorphousization inducing agent,

(7) the solid preparation according to (6), wherein the amorphousization inducing agent is 1 or 2 or more selected from the group consisting of amino acid or a salt thereof, aspartame, erythorbic acid or a salt thereof, ascorbic acid or a salt thereof, stearic acid ester, aminoethylsulfonic acid, inositol, ethylurea, citric acid or a salt thereof, glycyrrhizic acid or a salt thereof, gluconic acid or a salt thereof, creatinine, salicylic acid or a salt thereof, tartaric acid or a salt thereof, succinic acid or a salt thereof, calcium acetate, saccharine sodium, aluminum hydroxide, sorbic acid or a salt thereof, dehydroacetic acid or a salt thereof, sodium thiomalate, nicotinic acid amide, urea, fumaric acid or a salt thereof, macrogols, maltose, maltol, mannitol, meglumine, sodium desoxycholate and phosphatidylcholine,

(8) the solid preparation according to (6) or (7), wherein the amorphousization inducing agent is urea and/or saccharine sodium,

(9) the solid preparation according to any one of (6) to (8), wherein a content of the amorphousization inducing agent in the preparation is less than 8% by weight,

(10) the solid preparation according to (9), wherein a content of the amorphousization inducing agent in the preparation is 0.1 to 6% by weight,

(11) the solid preparation according to (10), wherein a content of the amorphousization inducing agent in the preparation is 2 to 4% by weight,

(12) the solid preparation according to any one of (1) to (11), wherein a content of the compound represented by the formula (I), a prodrug thereof, a pharmaceutically acceptable salt thereof or a solvate thereof in the preparation is 5 to 45% by weight,

(13) the solid preparation according to (12), wherein a content of the compound represented by the formula (I), a prodrug thereof, a pharmaceutically acceptable salt thereof or a solvate thereof in the preparation is 10 to 30% by weight,

(14) the solid preparation according to any one of (1) to (13), wherein the compound represented by the formula (I) in the preparation is trans-N-(5-trifluoromethylpyridin-2-yl)-4-(tertiarybutylsulfonylamino)cycloh exanecarboxamide,

(15) the solid preparation according to (14), which contains 5 to 45% by weight of trans-N-(5-trifluoromethylpyridin-2-yl)-4-(tertiarybutylsulfanylamino)cycloh exanecarboxamide, 40% by weight or more of hydroxypropylmethylcellulose phthalate and/or hydroxypropylmethylcellulose acetate succinate,

(16) the solid preparation according to (14), which contains 10 to 30% by weight of trans-N-(5-trifluoromethylpyridin-2-yl)-4-(tertiarybutylsulfonylamino)cycloh exanecarboxamide, 70 to 90% by weight or more of hydroxypropylmethylcellulose phthalate and/or hydroxypropylmethylcellulose acetate succinate,

(17) the solid preparation according to (14), which contains 10 to 20% by weight of trans-N-(5-trifluoromethylpyridin-2-yl)-4-(tertiarybutylsulfonylamino)cycloh exanecarboxamide, 80 to 90% by weight or more of hydroxypropylmethylcellulose phthalate and/or hydroxypropylmethylcellulose acetate succinate,

(18) the solid preparation according to (14), which contains 5 to 45% by weight of trans-N-(5-trifluoromethylpyridin-2-yl)-4-(tertiarybutylsulfonylamino)cycloh exanecarboxamide, 40% by weight or more of hydroxypropylmethylcellulose phthalate and/or hydroxypropylmethylcellulose acetate succinate, and less than 8% by weight of urea and/or saccharin sodium,

(19) the solid preparation according to (14), which contains 10 to 30% by weight of trans-N-(5-trifluoromethylpyridin-2-yl)-4-(tertiarybutylsulfonylamino)cycloh exanecarboxamide, 66 to 88% by weight or more of hydroxypropylmethylcellulose phthalate and/or hydroxypropylmethylcellulose acetate succinate, and 2 to 4% by weight of urea and/or saccharin sodium,

(20) the solid preparation according to (14), which contains 10 to 15% by weight of trans-N-(5-trifluoromethylpyridin-2-yl)-4-(tertiarybutylsulfonylamino)cycloh exanecarboxamide, 81 to 88 % by weight or more of hydroxypropylmethylcellulose phthalate and/or hydroxypropylmethylcellulose acetate succinate, and 2 to 4% by weight of urea and/or saccharin sodium,

(21) the solid preparation according to any one of (1) to (5), and (15) to (17), which does not contain the amorphousization inducing agent,

(22) the solid preparation according to any one of (1) to (21), which is a solid dispersion,

(23) a process for producing the solid preparation as defined in any one of (1) to (22), comprising using a spray drying method,

(24) a method of improving solubility of a NPYY5 receptor antagonist, comprising adding an amorphous stabilizer to a solid preparation containing the NPYY5 receptor antagonist, and

(25) a method of improving solubility of a NPYY5 receptor antagonist, comprising adding an amorphousization inducing agent to a solid preparation containing the NPYY5 receptor antagonist and an amorphous stabilizer.

[Effects of the invention]

The solid preparation of the present invention can increase dissolution of the NPYY5 receptor antagonist from the preparation. In addition, preferably, the solid preparation can maintain the amorphous state for a long period of time.

[Brief description of the drawings]

[Fig.1] An X-ray diffraction pattern of preparations having different contents of S-2367 (amorphous stabilizer: HPMCAS).
[Fig.2] Dissolution behavior of preparations having different contents of S-2367 (HPMCAS).
[Fig.3] An X-ray diffraction pattern of preparations having a content of S-2367 of 10% by weight, and different contents of urea (HPMCAS).
[Fig.4] An X-ray diffraction pattern of preparations having a content of S-2367 of 20% by weight, and different contents of urea (HPMCAS).
[Fig.5] An X-ray diffraction pattern of preparations having a content of S-2367 of 30% by weight, and different contents of urea (HPMCAS).
[Fig.6] An X-ray diffraction pattern of preparations having a content of S-2367 of 50% by weight, and different contents of urea (HPMCAS).
[Fig.7] Dissolution behavior of preparations having a content of S-2367 of 10% by weight, and different contents of urea (HPMCAS).
[Fig.8] Dissolution behavior of preparations having a content of S-2367 of 20% by weight, and different contents of urea (HPMCAS).
[Fig.9] Dissolution behavior of preparations having a content of S-2367 of 30% by weight, and different contents of urea (HPMCAS).
[Fig.10] Dissolution behavior of preparations having a content of S-2367 of 50% by weight, and different contents of urea (HPMCAS).
[Fig.11] An X-ray diffraction pattern of preparations having a content of S-2367 of 20% by weight, and different contents of urea (60°C, one week storage with time, HPMCAS).
[Fig.12] An X-ray diffraction pattern of preparations having a content of S-2367 of 20% by weight, and different contents of saccharine sodium (HPMCAS).
[Fig.13] Dissolution behavior of preparations having a content of S-2367 of 20% by weight, and different contents of saccharine sodium (HPMCAS). [Fig.14] An X-ray diffraction pattern of preparations having different contents of S-2367 (amorphous stabilizer: HPMCP).
[Fig.15] Dissolution behavior of preparations having different contents of S-2367 (HPMCP).
[Fig.16] An X-ray diffraction pattern of preparations having a content of S-2367 of 15% by weight, and different contents of urea (HPMCP).
[Fig.17] Dissolution behavior of preparations having a content of S-2367 of 15% by weight, and different contents of urea (HPMCP).
[Fig.18] An X-ray diffraction pattern of preparations having a content of S-2367 of 15 to 50% by weight, and a content of urea of 4% by weight (immediately after production, HPMCP).
[Fig.19] An X-ray diffraction pattern of preparations having a content of S-2367 of 15 to 50% by weight, and a content of urea of 4% by weight (60°C, one week storage, HPMCP).

[Best mode for carrying out the invention]

The NPYY5 receptor antagonist used in the present invention is preferably a compound represented by the formula (I), a prodrug thereof, a pharmaceutically acceptable salt thereof, or a solvate thereof, and is described in WO 01/37826 International Publication Pamphlet, and WO 03/076374 International Publication Pamphlet.
Herein, "halogen" includes fluorine, chlorine, bromine and iodine. Particularly, fluorine and chlorine are preferable.
"Lower alkyl" includes straight or branched alkyl of a carbon number of 1 to 10, and examples include methyl, ethyl, n-propyl, isopropyl, n-butyl, isobutyl, sec-butyl, tert-butyl, n-pentyl, isopentyl, neopentyl, hexyl, isohexyl, n-heptyl, isoheptyl, n-octyl, isooctyl, n-nonyl and n-decyl.
"Lower alkyl" in $R^1$ is preferably a carbon number of 3 to 10, further preferably a carbon number of 3 to 6, and most preferably isopropyl or t-butyl.
"Lower alkyl" in other cases is preferably a carbon number of 1 to 6, and further preferably a carbon number of 1 to 4.
Examples of a substituent of "lower alkyl optionally having a substituent" in Z include: (1) halogen; (2) cyano; and (3) (i)

hydroxy, (ii) lower alkoxy, (iii) mercapto, (iv) lower alkylthio, (v) acyl, (vi) acyloxy, (vii) carboxy, (viii) lower alkoxycarbonyl, (ix) imino, (x) carbamoyl, (xi) thiocarbamoyl, (xii) lower alkylcarbamoyl (xiii) lower alkylthiocarbamoyl, (xiv) amino, (xv) lower alkylamino or (xvi) heterocyclic carbonyl, each optionally being substituted with one or more replaceable groups selected from a substituent group β defined below.

Examples of a substituent of "lower alkyl optionally having a substituent "in the case other than Z (e.g., the case in $R^1$) include one or more groups selected from a substituent group β, and an arbitrary position may be substituted with these substituents.

The substituent group β is a group consisting of halogen, optionally protected hydroxyl, mercapto, lower alkoxy, lower alkenyl, amino, lower alkylamino, lower alkoxycarbonylamino, lower alkylthio, acyl, carboxy, lower alkoxycarbonyl, carbamoyl, cyano, cycloalkyl, phenyl, phenoxy, lower alkylphenyl, lower alkoxyphenyl, halogenophenyl, naphthyl and a heterocyclic group.

A lower alkyl part of "lower alkoxy," "lower alkoxycarbonyl," "lower alkoxycarbonyl lower alkyl," "lower alkylphenyl," "lower alkoxyphenyl," "lower alkylcarbamoyl," "lower alkylthiocarbamoyl," "lower alkylamino," "halogeno lower alkyl," "hydroxy lower alkyl," "phenyl lower alkoxy," "lower alkylthio," "phenyl lower alkylthio," "lower alkoxycarbonylamino," "lower alkoxycarbonyl lower alkenyl," "lower alkylsulfinyl," "lower alkylsufonyl," "aryl lower alkoxycarbonyl," "lower alkylbenzoyl" and "lower alkoxybenzoyl" is the same as the "lower alkyl."

Examples of a substituent of "lower alkoxy optionally having a substituent" include one or more groups selected from a substituent group β, preferably phenyl, lower alkylphenyl, lower alkoxyphenyl, naphthyl or a heterocyclic group.

"Cycloalkyl" includes cyclic alkyl of a carbon number of 3 to 8, and preferably 5 or 6. Specifically, examples include cyclopropyl, cyclobutyl, cyclopentyl, cyclohexyl, cycloheptyl and cyclooctyl.

Examples of a substituent of "cycloalkyl optionally having a substituent" include one or more groups selected from a substituent group α, and any position may be substituted.

"Bicycloalkyl" includes a group obtained by removing one hydrogen from an aliphatic ring of a carbon number of 5 to 8 in which two rings share two or more atoms. Specifically, examples include bicyclo[2.1.0]pentyl, bicyclo[2.2.1]heptyl, bicyclo[2.2.2]octyl and bicyclo[3.2.1]octyl.

"Lower alkenyl" includes straight or branched alkenyl of a carbon number of 2 to 10, preferably a carbon number of 2 to 8, and further preferably a carbon number of 3 to 6, and having one or more double bonds at an arbitrary position. Specifically, examples include vinyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl and decenyl.

A "lower alkenyl" part in "lower alkoxycarbonyl lower alkenyl" is the same as the "lower alkenyl."

Examples of a substituent of "lower alkenyl optionally having a substituent" include halogen, lower alkoxy, lower alkenyl, amino, lower alkylamino, lower alkoxycarbonylamino, lower alkylthio, acyl, carboxy, lower alkoxycarbonyl, carbamoyl, cyano, cycloalkyl, phenyl, lower alkylphenyl, lower alkoxyphenyl, naphthyl and/or a heterocyclic group.

"Acyl" includes (1) straight or branched alkylcarbonyl or alkenylcarbonyl of a carbon number of 1 to 10, further preferably a carbon number of 1 to 6, most preferably a carbon number of 1 to 4, (2) cycloalkylcarbonyl of a carbon number of 4 to 9, preferably a carbon number of 4 to 7, and (3) arylcarbonyl of a carbon number of 7 to 11. Specifically, examples include formyl, acetyl, propionyl, butyryl, isobutyryl, valeryl, pivaloyl, hexanoyl, acryloyl, propioyl, methacryloyl, crotonoyl, cyclopropylcarbonyl, cyclohexylcarbonyl, cyclooctylcarbonyl and benzoyl.

An acyl part of "acyloxy" is as defined above.

"Cycloalkenyl" includes an entity having 1 or more double bonds at an arbitrary position in the cycloalkyl ring and, specifically, examples include cyclopropenyl, cyclobutenyl, cyclopentenyl, cyclohexenyl, and cyclohexadienyl.

Examples of the substituent of "cycloalkenyl optionally having a substituent" include one or more groups selected from a substituent group β.

Examples of a substituent of "amino optionally having a substituent" include a substituent group β, benzoyl optionally having a substituent and/or heterocyclic carbonyl optionally having a substituent (herein, the substituent is hydroxy, lower alkyl, lower alkoxy and/or lower alkylthio."

"Aryl" is a monocyclic or polycyclic aromatic carbocyclic group, and includes phenyl, naphthyl, anthryl and phenanthryl. In addition, the aryl also includes aryl condensed with other non-aromatic hydrocarbon cyclic group and, specifically, examples include indanyl, indenyl, biphenylyl, acenaphthyl, tetrahydronaphthyl and fluorenyl. Particularly, phenyl is preferable.

An aryl part of "aryl lower alkoxycarbonyl" is the same.

"Aryl optionally having a substituent" and "phenyl optionally having a substituent" in Z include "aryl" or "phenyl" optionally substituted with a substituent group α, lower alkyl optionally substituted with one or more replaceable groups selected from a substituent group α, or the like.

Examples of a substituent of "aryl optionally having a substituent" and "phenyl optionally having a substituent" other than Z include one or more groups selected from a substituent group β.

"Hydrocarbon cyclic group" includes the "cycloalkyl," the "cycloalkenyl," the "bicycloalkyl" and the "aryl."

"Non-aromatic hydrocarbon cyclic group" includes the "cycloalkyl," the "cycloalkenyl" and the "bicycloalkyl."

"Hydrocarbon cyclic group optionally having a substituent" includes the "cycloalkyl optionally having a substituent," the "cycloalkenyl optionally having a substituent," the "bicycloalkyl optionally having a substituent" and the "aryl optionally having a substituent."

"Heterocyclic group" includes a heterocycle having one or more heteroatoms arbitrarily selected from O, S and N in a ring and, specifically, examples include 5- to 6-membered heteroaryls such as pyrrolyl, imidazolyl, pyrazolyl, pyridyl, pyridazinyl, pyrimidinyl, pyrazinyl, triazolyl, triazinyl, tetrazolyl, isoxazolyl, oxazolyl, oxadiazolyl, isothiazolyl, thiazolyl, thiadiazolyl, furyl and thienyl; bicyclic condensed heterocyclic groups such as indolyl, isoindolyl, indazolyl, indolizinyl, indolinyl, isoindolinyl, quinolyl, isoquinolyl, cinnolinyl, phthalazinyl, quinazolinyl, naphthyridinyl, quinoxalinyl, purinyl, pteridinyl, benzopyranyl, benzimidazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, imidazopyridyl, triazolopyridyl, imidazothiazolyl, pyrazinopyridazinyl, quinazolinyl, quinolyl, isoquinolyl, naphthyridinyl, dihydropyridyl, tetrahydroquinolyl, tetrahydrobenzothienyl and the like; tricyclic condensed heterocyclic groups such as carbazolyl, acridinyl, xanthenyl, phenothiazinyl, phenoxathiinyl, phenoxadinyl, dibenzofuryl and the like; non-aromatic heterocyclic groups such as dioxanyl, thiiranyl, oxiranyl, oxathiolanyl, azetidinyl, thianyl, pyrrolidinyl, pyrrolinyl, imidazolidinyl, imidazolinyl, pyrazolidinyl, pyrazolinyl, piperidyl, pyrazinyl, morpholinyl, morpholino, thiomorpholinyl, thiomorpholino, dihydropyridyl, tetrahydrofuryl, tetrahydropyranyl, tetrahydrothiazolyl, tetrahydroisothiazolyl and the like.

A condensed heterocyclic group condensed with a ring other than a heterocycle (e.g. benzothiazolyl and the like) may have a bond on any ring.

A substituent of "heterocyclic group optionally having a substituent" is the same substituent as that of "acryl optionally having a substituent."

A heterocyclic group part of "heterocyclic carbonyl," "heterocyclic oxy," "heterocyclic thio" and "heterocyclic substituted phenyl" is the same as the "heterocyclic group."

"Lower alkylene" includes a divalent group in which 1 to 6, preferably 2 to 6, further preferably 3 to 6 methylenes are continuous and, specifically, examples include methylene, ethylene, trimethylene, tetramethylene, pentamethylene and hexamethylene. Particularly preferable is tetramethylene.

A lower alkylene part of "lower alkylenedioxy" is the same as the "lower alkylene," preferably methylenedioxy or ethylenedioxy.

"Lower alkenylene" includes a divalent group in which 2 to 6, preferably 3 to 6, further preferably 4 to 5 methylenes are continuous, and at least one carbon-carbon bond is a double bond.

"Cycloalkylene" is a divalent group obtained by removing one hydrogen atom from the "cycloalkyl." In "cycloalkylene" in X, 1,4-cyclohexanediyl is preferable.

"Cycloalkenylene" includes a group having at least one double bond in a ring of the cycloalkylene.

"Bicycloalkylene" includes a group obtained by further removing one hydrogen from the "bicycloalkyl." Specifically, examples include bicyclo[2.1.0]pentylene, bicyclo[2.2.1]heptylene, bicyclo[2.2.2]octylene, bicyclo[3.2.1]octylene and the like.

"Heterocyclic diyl" includes a divalent group obtained by removing one hydrogen atom from the "heterocyclic group." Preferable are piperidinediyl, piperazinediyl, pyridinediyl, pyrimidinediyl, pyrazinediyl, pyrrolidinediyl, or pyrrolediyl, more preferable is piperidinediyl.

"Arylene" includes a divalent group obtained by removing one hydrogen atom from the "aryl." Preferable is phenylene.

"Heteroarylene" includes an entity having aromatic property among the "heterocyclic diyl." Specifically, examples include pyrrolediyl, imidazolediyl, pyrazolediyl, pyridinediyl, pyridazinediyl, pyrimidinediyl, pyrazinediyl, triazolediyl, triazinediyl, isoxazolediyl, oxazolediyl, oxadiazolediyl, isothiazolediyl, thiazolediyl, thiadiazolediyl, furandiyl and thiophenediyl.

Examples of a substituent of "lower alkylene optionally having a substituent," "lower alkenylene optionally having a substituent," "cycloalkylene optionally having a substituent," "cyclohexylene optionally having a substituent," "bicycloalkylene optionally having a substituent," "cycloalkenylene optionally having a substituent," "phenylene optionally having a substituent," "heterocyclic diyl optionally having a substituent" and "piperidinylene optionally having a substituent" include one or more replaceable groups selected from a substituent group β, preferably halogen, hydroxy, lower alkyl, halogeno lower alkyl, lower alkoxy, amino, lower alkylamino, acyl, carboxy or lower alkoxycarbonyl. An arbitrary position may be substituted with these groups.

The compound of the present invention includes a pharmaceutically acceptable salt which can be produced, of each compound. Examples of the "pharmaceutically acceptable salt" include salts of inorganic acids such as hydrochloric acid, sulfuric acid, nitric acid or phosphoric acid; salts of organic acids such as paratoluenesulfonic acid, methanesulfonic acid, oxalic acid or citric acid; salts of organic bases such as ammonium, trimethylammonium or triethylammonium; salts of alkali metals such as sodium or potassium; and salts of alkaline earth metals such as calcium or magnesium.

The compound of the present invention includes a solvate thereof, and corresponds to the compound (I). Preferable is a hydrate, and one molecule of the compound of the present invention may be coordinated with an arbitrary number of water molecules.

In addition, the compound of the present invention includes a prodrug thereof. A prodrug is a derivative of the compound

of the present invention having a group which can be chemically or metabolically degraded, and is a compound which serves as a pharmaceutically active compound of the present invention in vivo by solvolysis, or under physiological condition. A method of selecting a suitable prodrug derivative and a process for producing a suitable prodrug derivative are described, for example, in Design of Prodrugs, Elsevier, Amsterdam 1985.
For example, when the compound (I) of the present invention has carboxy, a prodrug such as an ester derivative produced by reacting carboxy of the compound (I) and a suitable alcohol, and an amide derivative produced by reacting carboxy of the compound (I) and a suitable amine is exemplified.
For example, when the compound (I) of the present invention has hydroxy, a prodrug such as an acyloxy derivative produced by reacting hydroxy of the compound (I) and suitable acyl halide or suitable acid anhydride is exemplified.
For example, when the compound (I) of the present invention has amino, a prodrug such as an amide derivative produced by reacting amino of the compound (I) and suitable acid halide or suitable mixed acid anhydride is exemplified.
When the compound (I) of the present invention has an asymmetric carbon atom, racemate, enantiomeric pairs and all steric isomers (geometrical isomer, epimer, enantiomer and the like) are included. In addition, when the compound (I) of the present invention has a double bond, and an E isomer and a Z isomer can be present, both of them are included.
In addition, when X is cycloalkylene, both a cis isomer and a trans isomer are included.
Examples of the compound represented by the formula (I) include preferably trans-N-(4-((2S, 6R)-2,6-dimethylmorpholino)phenyl)-4-(tertiarybutylsulfonylamino)cyclohex anecarboxamide, trans-N-(6-(5,6-dihydropyridin-1(2H)-yl)pyridin-3-yl)-4-(tertiarybutylsulfony lamino)cyclohexanecarboxamide, trans-N-(6-(4-trifluoromethyl)phenyl)pyridin-3-yl)-4-(tertiarybutylsulfonyla mino)cyclohexanecarboxamide, trans-N-(6-fluorobenzo[d]thiazol-2-yl)-4-(tertiarybutylsulfonylamino)cyclohe xanecarboxamide, trans-N-(5-trifluoromethylpyridin-2-yl)-4-(tertiarybutylsulfonylamino)cycloh exanecarboxamide (S-2367), and the following compounds.

[Chemical formula 6]

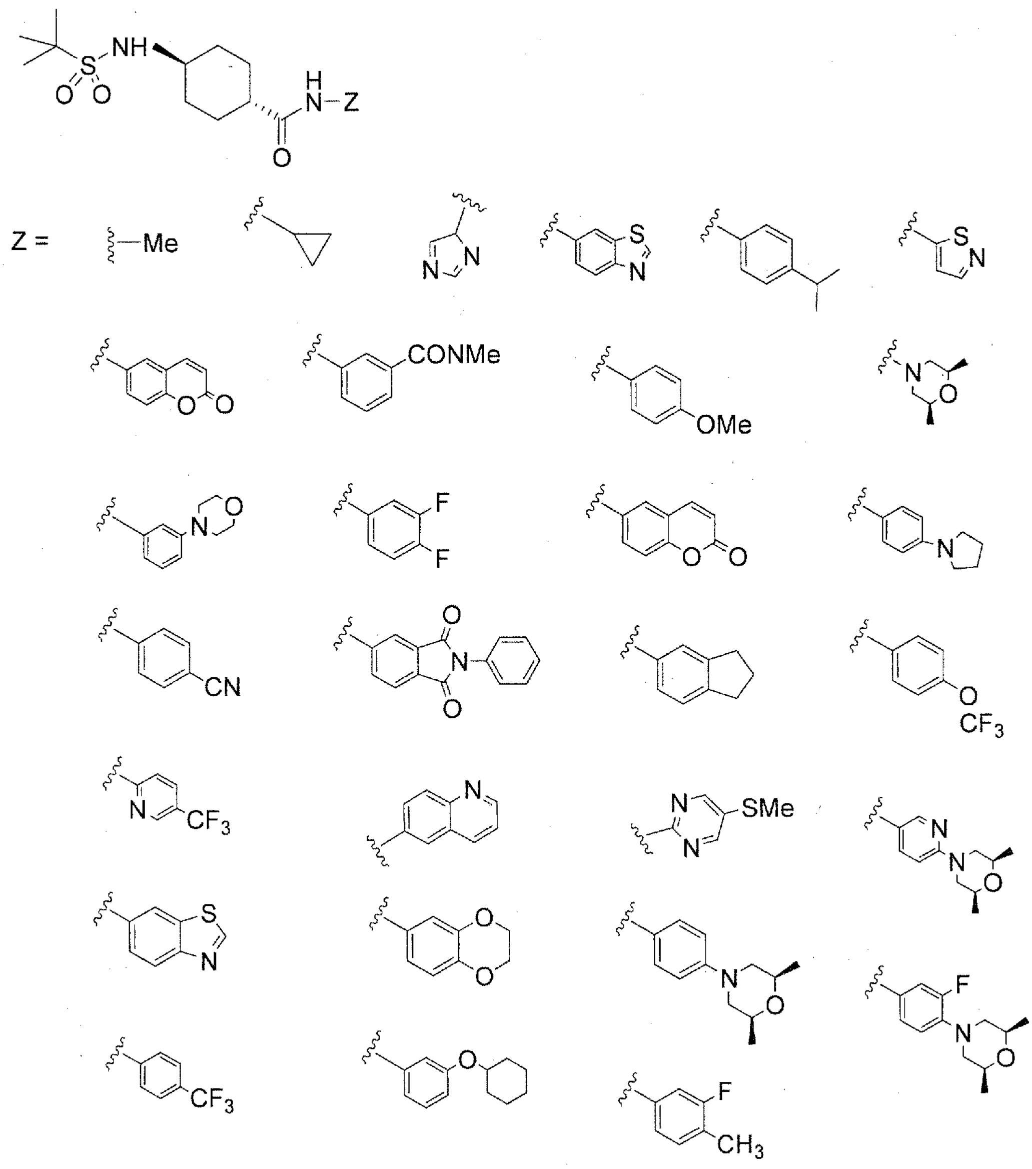

Z =

A particularly preferable compound is trans-N-(5-trifluoromethylpyridin-2-yl)-4-(tertiarybutylsulfonylamino)cycloh exanecarboxamide (S-2367). The compound is a crystal under a room temperature. In addition, solubility in water is very low (Japanese Pharmacopoeia, Dissolution Test, Second Solution (37°C); about 3.5 μg/mL).

A content of the NPYY5 receptor antagonist in the preparation of the present invention may be preferably an amount at which almost all amount of a drug can be dissolved out from the preparation. The content is usually 5 to 45% by weight, preferably 5 to 40% by weight, more preferably 7.5 to 40% by weight, further preferably 10 to 30% by weight, and particularly preferably 10 to 20% by weight based on a total amount of the preparation. When the content is less than this amount, there is a possibility that sufficient drug efficacy is not obtained and, when the content is more than this amount, there is a possibility that solubility cannot be sufficiently improved.

The NPYY5 receptor antagonist of the present invention is not particularly limited as far as it is a drug which can be orally administered, but is preferably a drug having low solubility in water, so-called poorly water-soluble drug. Herein, solubility of a drug in water refers to solubility of a drug at 37°C in any of a buffer and water having a pH considerable as the environment in a digestive tract in a range of 1 to 8, representatively, Japanese Pharmacopoeia, Disintegration Test Solution, First Solution, Disintegration Test Solution, Second Solution, and water, and examples include 100 μg/mL or less, further 50 μg/mL or less, and further 10 μg/mL or less.

The amorphous stabilizer preferably stabilizes the amorphous state by swinging a crystal structure of a poorly water-soluble drug with an amorphousization inducing agent and, thereafter, interacting with a fluctuating stage of a crystal lattice. However, an amorphous stabilizer include the one which itself can alone bring a crystal of the compounds into the amorphous state without blending the amorphousization inducing agent. Specifically, examples include polyvinylpyrrolidone, celluloses, crosslinked polyvinylpyrrolidone, polyvinyl alcohol, polyvinyl acetate, vinyl alcohol/vinyl acetate copolymer, ethylene/vinyl acetate copolymer, polyethylene oxide derivative (e.g. polyethylene glycol, polyoxyethylene polyoxypropylene cetyl ether, polyoxyethylene alkyl ether, polyoxyethylene octyl phenyl ether, polyoxyethylene oleyl amine, polyoxyethylene oleyl ether, polyoxyethylene oleyl ether sodium phosphate, polyoxyethylene hydrohenated castor oil, polyoxyethylene stearyl ether, polyoxyethylene stearyl ether phosphoric acid, polyoxyethylene cetyl ether, polyoxyethylene cetyl ether sodium phosphate, polyoxyethylene sorbit beeswax, polyoxyethylene nonyl phenyl ether, polyoxyethylene castor oil, polyoxyethylene behenyl ether, polyoxyethylene polyoxypropylene glycol, polyoxyethylene polyoxypropylene cetyl ether, polyoxyethylene lauryl ether, polyoxyethylene lanolin, polysorbate 40, polysorbate 60, polysorbate 65, polysorbate 80 and the like), sodium polystyrenesulfonate, gelatin, starch, dextran, agar, sodium alginate, pectin, pullulan, xanthan gum, acacia, chondroitin sulfate or a sodium salt thereof, hyaluronic acid, chitin, chitosan, α, β or γ-cyclodextrin, alginic acid derivative, acryl resins, polyvinylacetal diethylaminoacetate, silicon dioxide, carrageenan and aluminum hydroxide.

Preferably, examples of the amorphous stabilizer include polyvinylpyrrolidone, celluloses, polyvinyl alcohol, polyvinyl acetate, gelatin, agar, sodium alginate, pectin, pullulan, xanthan gum, acacia, chondroitin sulfate, hyaluronic acid and carrageenan.

More preferably, examples of the amorphous stabilizer include hydroxypropylcellulose, hydroxypropylmethylcellulose, hydroxypropylmethylcellulose phthalate (HPMCP), hydroxypropylmethylcellulose acetate succinate (HPMCAS) and carboxymethylcellulose sodium.

Particularly preferably, examples of the amorphous stabilizer include HPMCP and/or HPMCAS.

As hydroxypropylmethylcellulose phthalate (HPMCP, another name:
hypromellose phthalic acid ester), those listed in Japanese Pharmacopoeia, 15$^{th}$ edition may be used, preferable is substituted type 200731 (carboxybenzoyl group is 27.0 to 35.0%) and substituted type 220824 (carboxybenzoyl group is 21.0 to 27.0%), and more preferable is substituted type 220731. Specifically, it is HPMCP HP-55, HP-55S, and HP-50 (manufactured by Shin-Etsu Chemical Co., Ltd., Samsung Fine Chemicals), preferably HPMCP HP-55.

As hydroxypropylmethylcellulose acetate succinate (HPMCAS), those described in Japanese Pharmaceutical Excipients may be used, and examples include preferably those that can be dissolved in water having a pH higher than 5.5, specifically Shin-Etsu AQOAT HPMCAS-LF (which is dissolved in McIlvaine's Buffer Solution, at a pH 5.5 or higher), HPMCAS-MF (which is dissolved in the same solution at a pH 6.0 or higher), and HPMCAS-HF (which is dissolved in the same solution at a pH 6.8 or higher) (all manufactured by Shin-Etsu Chemical Co., Ltd.), and more preferably Shin-Etsu AQOAT HPMCAS-LF.

A content of the amorphous stabilizer in the preparation of the present invention is preferably a content at which almost all amount of the NPYY5 receptor antagonist can be dissolved out from the preparation. The content is usually 40% by weight or more, preferably 60 to 95% by weight, more preferably 60 to 92.5% by weight, further preferably 70 to 90% by weight, and particularly preferably 80 to 90% by weight based on a total amount of the preparation. When the content is less than this amount, since the effect of suppressing precipitation of a water-soluble polymer crystal is decreased, there is a possibility that a drug is crystallized during a production process, and an amorphous preparation cannot be obtained and, when the content is more than this amount, there is a possibility that solubility cannot be improved.

One of preferable aspects of the present preparation is such that (1) usually, the NPYY5 receptor antagonist is 5 to 45% by weight, and the amorphous stabilizer is 40% by weight or more based on a total amount of the preparation. (2) Preferably, the NPYY5 receptor antagonist is 5 to 40% by weight, and the amorphous stabilizer is 60 to 95% by weight. (3) More preferably, the NPYY5 receptor antagonist is 7.5 to 40% by weight, and the amorphous stabilizer is 60 to 92.5% by weight. (4) Further preferably, the NPYY5 receptor antagonist is 10 to 30% by weight, and the amorphous stabilizer is 70 to 90% by weight. (5) Particularly preferably, the NPYY5 receptor antagonist is 10 to 20% by weight, and the amorphous stabilizer is 80 to 90% by weight.

One of preferable aspects of the present preparation is such that (1) usually, the NPYY5 receptor antagonist is 5 to 45% by weight, and HPMCP and/or HPMCAS is 40% by weight or more based on a total amount of the preparation. (2) Preferably, the NPYY5 receptor antagonist is 5 to 40% by weight, and HPMCP and/or HPMCAS is 60 to 95% by weight. (3) More preferably, the NPYY5 receptor antagonist is 7.5 to 40% by weight, and HPMCP and/or HPMCAS is 60 to 92.5% by weight. (4) Further preferably, the NPYY5 receptor antagonist is 10 to 30% by weight, and HPMCP and/or HPMCAS is 70 to 90% by weight. (5) Particularly preferably, the NPYY5 receptor antagonist is 10 to 20% by weight, and HPMCP and/or HPMCAS is 80 to 90% by weight.

One of preferable aspects of the present preparation is such that (1) usually, S-2367 is 5 to 45% by weight, and HPMCP and/or HPMCAS is 40% by weight or more based on a total amount of the preparation. (2) Preferably, S-2367 is 5 to 40% by weight, and HPMCP and/or HPMCAS is 60 to 95% by weight. (3) More preferably, S-2367 is 7.5

to 40% by weight, and HPMCP and/or HPMCAS is 60 to 92.5% by weight. (4) Further preferably, S-2367 is 10 to 30% by weight, and HPMCP and/or HPMCAS is 70 to 90% by weight. (5) Particularly preferably, S-2367 is 10 to 20% by weight, and HPMCP and/or HPMCAS is 80 to 90% by weight.

The amorphousization inducing agent used in the present invention is a compound which changes a crystal lattice energy of a poorly water-soluble drug towards a low energy direction, and has function/nature of increasing fluctuation of a crystal lattice at the same temperature. Specifically, examples include amino acid or a salt thereof (aspartic acid and sodium salt, magnesium salt thereof and the like, glycine, alanine, glutamic acids, glutamic acid hydrochloride and the like), aspartame, erythorbic acid or a salt thereof, ascorbic acid or a salt thereof (sodium salt), stearic acid ester, aminoethylsulfonic acid, inositol, ethylurea, citric acid or a salt thereof (salt of trisodium, disodium, dihydrogen sodium and the like, calcium salt and the like), glycyrrhizic acid or a salt thereof (sodium salt of trisodium, disodium and the like), ammonium salt of diammonium, monoammonium and the like, potassium salt and the like), gluconic acid or a salt thereof (sodium salt, calcium salt, magnesium salt and the like), creatinine, salicylic acid or a salt thereof (sodium salt and the like), tartaric acid or a salt thereof (sodium salt, potassium/sodium salt, hydrogen/potassium salt and the like), succinic acid or a salt thereof (sodium salt of disodium, monosodium and the like), calcium acetate, saccharine sodium, aluminum hydroxide, sorbic acid or a salt thereof (potassium salt and the like), dehydroacetic acid or a salt thereof (sodium salt etc.), sodium thiomalate, nicotinic acid amide, urea, fumaric acid or a salt thereof (sodium salt and the like), macrogols, maltose, maltol, maleic acid, mannitol, meglumine, sodium desoxycholate and phosphatidylcholine.

Examples of the amorphousization inducing agent include preferably amino acid or a salt thereof (aspartic acid and sodium salt, magnesium salt thereof and the like, glycine, alanine, glutamic acids and glutamic acid hydrochloride and the like), ascorbic acid or a salt thereof (sodium salt etc.), stearic acid ester, aminoethylsulfonic acid, ethylurea, citric acid or a salt thereof (salt of trisodium, disodium, dihydrogen sodium and the like, calcium salt), glycyrrhizic acid or a salt thereof (sodium salt of trisodium, disodium and the like), ammonium salt of diammonium, monoammonium and the like, potassium salt and the like), creatinine, tartaric acid or a salt thereof (sodium salt, sodium/potassium salt, hydrogen/potassium salt and the like), succinic acid or a salt thereof (sodium salt of disodium, monosodium and the like), saccharine sodium, nicotinic acid amide, urea, fumaric acid or a salt thereof (sodium salt and the like), macrogols, maltose, maltol, mannitol, and meglumine.

Examples of the amorphousization inducing agent include more preferably amino acid or a salt thereof (aspartic acid and sodium salt, magnesium salt thereof and the like, glycine, alanine, glutamic acids and glutamic acid hydrochloride and the like), ethylurea, glycyrrhizic acid or a salt thereof (sodium salt of trisodium, disodium and the like), ammonium salt of diammonium, monoammonium and the like, potassium salt and the like), tartaric acid or a salt thereof (sodium salt, sodium/potassium salt, hydrogen/potassium salt and the like), succinic acid or a salt thereof (sodium salt of disodium, monosodium and the like), saccharine sodium, nicotinic acid amide, urea, maltose, maltol, mannitol, and meglumine. Particularly preferable is urea and/or saccharine sodium.

When the preparation of the present invention contains the amorphousization inducing agent, a content of the amorphous stabilizer may be a content at which almost all amount of the NPYY5 receptor antagonist can be dissolved out from the preparation, and is usually 40% by weight or more, preferably 54 to 94.9% by weight, more preferably 55 to 92% by weight, and particularly preferably 66 to 88% by weight based on a total amount of the preparation. When the content is less than this amount, since the effect of suppressing precipitation of a water-soluble polymer is decreased, there is a possibility that a drug is crystallized during a production process, and an amorphous preparation is not obtained and, when the content is more than this amount, there is a possibility that solubility cannot be improved.

A content of the amorphousization inducing agent in the preparation of the present invention may be a content at which solubility of the NPYY5 receptor antagonist can be improved, and is usually less than 8% by weight, preferably 0.5 to 6% by weight, more preferably 0.5 to 5% by weight, and particularly preferably 2 to 4% by weight based on a total amount of the preparation. Two or more kinds of amorphousization inducing agents may be used and, when they are used, it is enough that a total amount of them is in a range of the content. When the total amount is less than this amount, there is a possibility that solubility of a drug cannot be increased and, when the total amount is more than this amount, there is a possibility that solubility of a drug is decreased, and a side effect of the amorphousization inducing agent is caused, and since the effect of suppressing precipitation of a crystal of the amorphous stabilizer (particularly, water-soluble polymer) during a production process is decreased, there is a great possibility that a drug is easily crystallized upon removal of a solvent, and it becomes difficult to obtain an amorphous preparation.

One of preferable content of the present preparation is such that (1) usually, the NPYY5 receptor antagonist is 5 to 45% by weight, and the amorphousization inducing agent is less than 8% by weight, based on a total amount of the preparation. (2) Preferably, the NPYY5 receptor antagonist is 5 to 40% by weight, and the amorphousization inducing agent is 0.1 to 6% by weight. (3) More preferably, the NPYY5 receptor antagonist is 7.5 to 40% by weight, and the amorphousization inducing agent is 0.5 to 5% by weight. (4) Further preferably, the NPYY5 receptor antagonist is 10 to 30% by weight, and the amorphousization inducing agent is 2 to 4% by weight. (5) Particularly preferably, the NPYY5 receptor antagonist is 10 to 20% by weight, and the amorphousization inducing agent is 2 to 4% by weight. (6) Considerably preferably, the NPYY5 receptor antagonist is 10 to 15% by weight, and the amorphousization inducing agent is 2 to 4%

by weight.

One of preferable content of the present preparation is such that (1) usually, the NPYY5 receptor antagonist is 5 to 45% by weight, the amorphous stabilizer is 40 % by weight or more, and the amorphousization inducing agent is less than 8% by weight, based on a total amount of the preparation. (2)Preferably, the NPYY5 receptor antagonist is 5 to 40% by weight, the amorphous stabilizer is 54 to 94.9 % by weight, and the amorphousization inducing agent is 0.1 to 6% by weight. (3)More preferably, the NPYY5 receptor antagonist is 7.5 to 40% by weight, the amorphous stabilizer is 55 to 92 % by weight, and the amorphousization inducing agent is 0.5 to 5% by weight. (4)Further preferably, the NPYY5 receptor antagonist is 10 to 30% by weight, the amorphous stabilizer is 66 to 88 % by weight, and amorphousization inducing agent is 2 to 4% by weight. (5)
Particularly preferably, the NPYY5 receptor antagonist is 10 to 20% by weight, the amorphous stabilizer is 76 to 88 % by weight, and the amorphousization inducing agent is 2 to 4% by weight. (6) Considerably preferably, the NPYY5 receptor antagonist is 10 to 15% by weight, the amorphous stabilizer is 81 to 88 % by weight, and the amorphousization inducing agent is 2 to 4% by weight.

A preferable combination of the amorphousization inducing agent and the amorphous stabilizer is (1) urea, saccharine sodium and HPMCAS, (2) urea and HPMCAS, (3) saccharine sodium and HPMCAS, (4) urea, saccharine sodium and HPMCP, (5) urea and HPMCP, and (6) saccharine sodium and HPMCP.

One of preferable content of the present preparation is such that (1) usually, the NPYY5 receptor antagonist is 5 to 45% by weight, and HPMCP and/or HPMCAS is 40% by weight or more, and urea and/or saccharine sodium is less than 8% by weight, based on a total amount of the preparation. (2) Preferably, the NPYY5 receptor antagonist is 5 to 40% by weight, HPMCP and/or HPMCAS is 54 to 94.9 % by weight, and urea and/or saccharine sodium is 0.1 to 6% by weight. (3) More preferably, the NPYY5 receptor antagonist is 7.5 to 40% by weight, HPMCP and/or HPMCAS is 55 to 92 % by weight, and urea and/or saccharine sodium is 0.5 to 5% by weight. (4) Further preferably, the NPYY5 receptor antagonist is 10 to 30% by weight, HPMCP and/or HPMCAS is 66 to 88 % by weight or more, and urea and/or saccharine sodium is less than 2 to 4% by weight. (5) Particularly preferably, the NPYY5 receptor antagonist is 10 to 20% by weight, HPMCP and/or HPMCAS is 76 to 88 % by weight, and urea and/or saccharine sodium is 2 to 4% by weight. (6) Considerably preferably, the NPYY5 receptor antagonist is 10 to 15% by weight, HPMCP and/or HPMCAS is 81 to 88 % by weight, and urea and/or saccharine sodium is 2 to 4% by weight.

One of preferable content of the present preparation is such that (1) usually, S-2367 is 5 to 45% by weight, HPMCP and/or HPMCAS is 40 % by weight or more, and urea and/or saccharine sodium is less than 8% by weight, based on a total amount of the preparation. (2) Preferably, S-2367 is 5 to 40% by weight, the HPMCP and/or HPMCAS is 54 to 94.9 % by weight or more, and urea and/or saccharine sodium is 0.1 to 6% by weight. (3) More preferably, S-2367 is 7.5 to 40% by weight, HPMCP and/or HPMCAS is 55 to 92 % by weight, and urea and/or saccharine sodium is 0.5 to 5% by weight. (4) Further preferably, S-2367 is 10 to 30% by weight, and HPMCP and/or HPMCAS is 66 to 88 % by weight, and urea and/or saccharine sodium is 2 to 4% by weight. (5) Particularly preferably, S-2367 is 10 to 20% by weight, HPMCP and/or HPMCAS is 76 to 88 % by weight, and urea and/or saccharine sodium is 2 to 4% by weight. (6) Considerably preferably, S-2367 is 10 to 15% by weight, HPMCP and/or HPMCAS is 81 to 88 % by weight, and urea and/or saccharine sodium is 2 to 4% by weight.

In the process for producing the present preparation, preferably, the NPYY5 receptor antagonist, the amorphous stabilizer and, optionally, the amorphousization inducing agent are dissolved in a solvent, the solvent is removed, and the resulting solid is ground into a suitable particle size. The solvent may be a solvent in which these raw materials are dissolved. A specific solvent is water, alcohol, acetone, halogenated carbon and a mixture thereof. In addition, as a method of removing the solvent, there are warming under reduced pressure and spray drying.

In the present invention, it was revealed that, upon formation of a solid dispersion, amounts of the amorphous stabilizer and the amorphousization inducing agent in the solid dispersion influence on solubility and stability of the NPYY5 receptor antagonist. In addition, improvement of solubility of the NPYY5 receptor antagonist in water was also performed by confirming the amorphousized state by powder X-ray analysis.

The present preparation is obtained in a powder form, a granule form, a mass form of a solid. Even when the present preparation is obtained in a mass form, grinding or the like can afford a powder. The powder can be also contained in a granule or a tablet. As a diluent, a binder, a lubricant and the like used in the granule or the tablet, those that have previously been used in pharmaceutical preparations can be used. Examples include diluents such as D-mannitol and the like, disintegrating agents such as carmellose calcium and the like, binders such as hydroxypropylcellulose and the like, lubricants such as magnesium stearate, coating agents such as hydroxypropylmethylcellulose and the like, and the like.

## Examples

The following Examples illustrate the present invention in more detail, but the present invention is not limited by these Examples at all.

(Process for producing preparation containing HPMCAS)

Trans-N-(5-trifluoropyridin-2-yl)-4-(tertiarybutylsulfonylamino)cyclo hexanecarboxamide (S-2367) was used as the NPYY5 receptor antagonist, urea (manufactured by Wako Pure Chemical Industries, Ltd.) or saccharine sodium (manufactured by Oriental Pharmaceutical and Synthetic Chemical Co., Ltd.) was used as the amorphousization inducing agent, and hydroxypropylmethylcellulose acetate succinate (HPMCAS-LF, manufactured by Shin-Etsu Chemical Co., Ltd.) was used as the amorphous stabilizer. In addition, S-2367 was produced based on the method described in WO 01/37826 International Publication Pamphlet and WO 03/076374 International Publication Pamphlet.
After a formulation amount shown in Table 1 of S-2367 was added to acetone to completely dissolve it, a formulation amount of HPMCAS was added to dissolve it. Then, a formulation amount of urea or saccharine sodium was added to ethanol to dissolve it. These solutions were mixed at a ratio of 2:1 when S-2367 was 10 to 20% by weight, or at a ratio of 92:8 in the case of 30 to 50% by weight, to produce a solution of S-2367, and then the solution was spray-dried at an exit temperature of 85°C into the powder state using a spray dryer, B-290/B-295 (manufactured by Buchi).

(Dissolution test method)

A solid dispersion powder having a content of S-2367 corresponding to 20 mg was subjected to the dissolution test according to the method defined in 15$^{th}$ revision, Japanese Pharmacopoeia. A concentration of S-2367 in a test solution was measured using an automatic sampling system (Autosampler W PAS-615 (manufactured by Toyama Sangyo Co., Ltd.), and spectrophotometer UV-1700 (manufactured by Shimadzu Corporation)).
The dissolution test condition is as follows:

- Test method: Japanese Pharmacopoeia, Second Method (paddle method, rotation rate 50 rpm
- Test solution: dissolution test condition, second solution, (900 mL, 37°C)
- Test solution collecting time: 0, 5, 10, 15, 20, 25, 30, 45, 60 minutes
- Detection wavelength: 243 nm
- Layer length: 5 mm

(Powder X-ray diffraction)

A powder X-ray diffraction pattern of the solid dispersion was investigated using a powder X-ray diffraction apparatus, RINT2000 (manufactured by Rigaku Corporation).

(Influence of content on dissolution property of NPYY5 receptor antagonist)

Preparations in which urea or saccharine sodium is not contained therein, and a content ratio of S-2367 and HPMCAS is as shown in Table 1 were produced by the aforementioned process. After conversion of these preparations into amorphous was confirmed, a dissolution test was performed by the aforementioned method.

[Table 1] (weight %)

| | Reference Example 1 | Reference Example 2 | Reference Example 3 | Comparative Example 1 |
|---|---|---|---|---|
| S-2367 | 10.0 | 20.0 | 30.0 | 50.0 |
| HPMCAS | 90.0 | 80.0 | 70.0 | 50.0 |
| Total | 100.0 | 100.0 | 100.0 | 100.0 |

(Results)

As shown in Fig.1, in any preparation, a peak of a crystal of S-2367 is not detected in X-ray diffraction, and it was revealed that S-2367 was converted into amorphous. On the other hand, a dissolution concentration of S-2367 was investigated with time and, as a result, as shown in Fig.2, as a content of HPMCAS was increased, a dissolution concentration was increased and, when HPMCAS was 70% by weight or more, solubility exceeded solubility of S-2367 (about 3.5 μg/mL).

(Influence of urea on dissolution property of NPYY5 receptor antagonist)

As shown in Tables 2 to 5, preparations containing 10 to 50% by weight of S-2367 and 2 to 12% by weight of urea were produced, conversion into amorphous was confirmed, and dissolution behavior was investigated.

[Table 2] (weight %)

| | Reference Example 1 | Example 1 |
|---|---|---|
| S-2367 | 10.0 | 10.0 |
| HPMCAS | 90.0 | 86.0 |
| Urea | - | 4.0 |
| Total | 100.0 | 100.0 |

[Table 3] (weight %)

| | Reference Example 2 | Example 2 | Example 3 | Comparative Example 2 | Comparative Example 3 |
|---|---|---|---|---|---|
| S-2367 | 20.0 | 20.0 | 20.0 | 20.0 | 20.0 |
| HPMCAS | 80.0 | 78.0 | 76.0 | 72.0 | 68.0 |
| Urea | - | 2.0 | 4.0 | 8.0 | 12.0 |
| Total | 100.0 | 100.0 | 100.0 | 100.0 | 100.0 |

[Table 4] (weight %)

| | Reference Example 3 | Example 4 |
|---|---|---|
| S-2367 | 30.0 | 30.0 |
| HPMCAS | 70.0 | 66.0 |
| Urea | - | 4.0 |
| Total | 100.0 | 100.0 |

[Table 5] (weight %)

| | Comparative Example 1 | Comparative Example 4 |
|---|---|---|
| S-2367 | 50.0 | 50.0 |
| HPMCAS | 50.0 | 46.0 |
| Urea | - | 4.0 |
| Total | 100.0 | 100.0 |

(Results)

Fig.3 to Fig.6 show results of X-ray diffraction of preparations having a content of S-2367 of 10, 20, 30 and 50% by weight. As a result, a peak of a crystal of S-2367 was not detected in any preparation, and it was revealed that S-2367 was converted into amorphous. Fig.7 to Fig.10 show results of investigation of a dissolution concentration of S-2367 with time, with respect to preparations having a content of S-2367 of 10, 20, 30 and 50% by weight. As a result, in the case of preparations where S-2367 is 10, 20, or 30 by weight, it was revealed that as a content of urea is increased, a dissolution rate is increased, but in the case of the preparation of 50% by weight, even when urea was added, a dissolution rate was hardly changed, and solubility in water was not higher than that of S-2367.

(Influence of urea on stability of NPYY5 receptor antagonist when preparation is stored with time)

A solid preparation containing 20% by weight of S-2367 (Reference Example 2, Examples 2 and 3, Comparative Examples 2 and 3) was stored with time at 60°C for one week, and powder X-ray analysis was performed to confirm crystallizability of S-2367.

(Results)

As shown in Fig. 11, in a preparation containing 0 to 4% by weight of urea (Reference Examples, Examples 2 and 3), after one week storage at 60 °C, a peak of a crystal of S-2367 was not detected in powder X-ray analysis, and it was revealed that S-2367 was converted into amorphous. To the contrary, in a preparation containing 8 or 12% by weight of urea (Comparative Examples 2 and 3), a main crystal peak (2θ = 16.9, 17.9°) of S-2367 was detected on a powder X-ray diffraction chart after one week storage at 60°C, and it was revealed that, in the case of a larger amount of urea, dissolution property is improved, but a solid dispersion is easily crystallized.

(Influence of saccharine sodium on dissolution property of NPYY5 receptor antagonist)

As shown in Table 6, preparations containing 20% by weight of S-2367, saccharine sodium and HPMCAS were produced, conversion into amorphous was confirmed, and dissolution behavior was investigated.

[Table 6] (weight %)

| | Reference Example 1 | Example 5 | Example 6 |
|---|---|---|---|
| S-2367 | 20.0 | 20.0 | 20.0 |
| HPMCAS | 80.0 | 76.0 | 70.0 |
| Saccharine sodium | - | 4.0 | 10.0 |
| Total | 100.0 | 100.0 | 100.0 |

(Results)

As shown in Fig.12, in powder X-ray analysis of any preparation, a peak of a crystal of S-2367 was not detected, and it was revealed that S-2367 was converted into amorphous. On the other hand, a dissolution concentration of S-2367 was investigated with time and, as a result, as shown in Fig.13, it was revealed that a dissolution rate is increased in a preparation containing saccharine sodium (Example 5 and 6) as compared with a preparation not containing saccharine sodium (Reference Example 2). In addition, a dissolution rate was increased in a preparation containing 4% by weight of saccharine sodium as compared with a preparation containing 10% by weight of saccharine sodium.

(Process for producing preparation containing HPMCP)

Trans-N-(5-trifluoromethylpyridin-2-yl)-4-(tertiarybutylsulfonylamin o)cyclohexanecarboxamide (S-2367) was used as the NPYY5 receptor antagonist, urea (manufactured by Wako Pure Chemical Industries, Ltd.) was used as the amorphousization inducing agent, and hydroxypropylmethylcellulose phthalate (grade: HP55, substituted type: 200731, manufactured by Samsung Fine Chemicals, HPMCP) was used as the amorphous stabilizer. In addition, S-2367 was produced based on the process described in WO 01/37826 International Publication Pamphlet, and WO 03/076374 International Publication Pamphlet. After a formulation amount shown in Table 7 of S-2367 was added to acetone to completely dissolve it, a formulation amount of HPMCP was added to dissolve it. Then, a formulation amount of urea was added to ethanol to dissolve it. These solutions were mixed at a ratio of 1:0 in the case of 0% by weight of urea, or at a ratio of 5.64:1 in the case of 4% by weight of urea, to produce a S-2367 solution, and the solution was spray-dried at an exit temperature of 80°C into the powder state using a spray dryer, B-290/B-295 (manufactured by Buchi).

(Dissolution test method)

A solid dispersion powder having a content of S-2367 corresponding to 20 mg was subjected to the dissolution test according to the method defined in 16$^{th}$ revision, Japanese Pharmacopoeia. A concentration of S-2367 in a test solution was measured using liquid chromatography (1100 Series manufactured by Agilent). The condition of the dissolution test and the condition of liquid chromatography (HPLC) are as follows.

- Condition of dissolution test
  Test method: Japanese Pharmacopoeia, Second Method, paddle method), rotation rate 50 rpm
  Test solution: second solution, (900 mL, 37°C)
  Test solution collecting time: 0, 5, 10, 15, 30, 60 minutes
  Drug concentration measuring method: After the collected solution was filtered with a filter having a pore diameter of 0.45 μm, the filtrate was diluted with methanol. A drug concentration in the diluted solution was measured by the HPLC method.
- HPLC condition
  Measuring wavelength: 242 nm
  Column: Capcell Pak C18 MG, 3 μm, 3.0 x 50 mm, Shiseido Co., Ltd. Column temperature: 35°C
  Mobile phase: methanol/water mixed solution (27 : 73) for HPLC
  Flow rate: about 0.6 mL/min
  Injection amount: 15 μL
  As a method of calculating a dissolution concentration, each numerical value was substituted into the following equation.

(Equation 1)

$$\text{Content of S-2367 (\%)} = W_S \times (A_T/A_S) \times (1/500) \times 100$$

$W_S$: amount of S-2367 standard (mg)
$A_S$: Peak area of standard solution
$A_T$: Peak area of sample solution
1/500: Dilution factor

(Powder X-ray diffraction)

A powder X-ray diffraction pattern of a solid dispersion was investigated using a powder X-ray diffraction apparatus, RINT III (manufactured by Rigaku Corporation).

(Influence of content on dissolution property of NPYY5 receptor antagonist)

Preparations in which urea or saccharine sodium is not contained in the preparation, and a content ratio of S-2367 and HPMCP is shown in Table 7, were produced by the aforementioned process. After conversion of these preparations into amorphous was confirmed, a dissolution test was performed by the aforementioned method.

[Table 7] (weight %)

| | Reference Example 4 | Reference Example 5 | Reference Example 6 |
|---|---|---|---|
| S-2367 | 10.0 | 15.0 | 20.0 |
| HPMCP | 90.0 | 85.0 | 80.0 |
| Total | 100.0 | 100.0 | 100.0 |

(Results)

As shown in Fig.14, in any preparation, a peak of a crystal of S-2367 is not detected in X-ray diffraction, and it was revealed that S-2367 was converted into amorphous. On the other hand, a dissolution concentration of S-2367 was investigated with time and, as a result, as shown in Fig.15, as a content of HPMCP was increased, a dissolution concentration was increased.

(Influence of urea on dissolution property of NPYY5 receptor antagonist)

As shown in Table 8, preparations containing 15% by weight of S-2367, and 0 to 4% by weight of urea were produced, conversion into amorphous was confirmed, and dissolution behavior was investigated.

[Table 8] (weight %)

| | Reference Example 5 | Example 7 |
|---|---|---|
| S-2367 | 15.0 | 15.0 |
| HPMCP | 85.0 | 81.0 |
| Urea | - | 4.0 |
| Total | 100.0 | 100.0 |

(Results)

Fig.16 shows results of X-ray diffraction of preparations having a content of S-2367 of 15% by weight, and containing 0 to 4% by weight of urea. As a result, in any preparation, a peak of a crystal of S-2367 was not detected, and it was revealed that S-2367 was converted into amorphous.
Fig.17 shows results of investigation of a dissolution concentration of S-2367 with time, with respect to preparations having a content of S-2367 of 15% by weight. As a result, it was revealed that when urea is blended at 4%, a dissolution rate of S-2367 is increased.

(Influence of urea on stability of NPYY5 receptor antagonist when preparation is stored with time)

Preparations shown in Table 9 were stored at 60°C for one week with time, powder X-ray analysis was performed, and crystallizability of S-2367 was confirmed.

[Table 9] (weight %)

| | Example 7 | Comparative Example 5 | Comparative Example 6 | Comparative Example 7 |
|---|---|---|---|---|
| S-2367 | 15.0 | 20.0 | 30.0 | 50.0 |
| HPMCP | 81.0 | 76.0 | 66.0 | 46.0 |
| Urea | 4.0 | 4.0 | 4.0 | 4.0 |
| Total | 100.0 | 100.0 | 100.0 | 100.0 |

(Results)

Fig.18 shows powder X-ray diffraction immediately after preparation production, and a crystal peak peculiar for S-2367 was not observed. Fig.19 shows powder X-ray diffraction after one week storage at 60°C, when S-2367 is 15% by weight, a crystal peak peculiar for S-2367 is not detected, and it was revealed that S-2367 was converted into amorphous. To the contrary, in the case of a preparation with 20% by weight or more of S-2367 blended therein (Comparative Examples 5, 6 and 7), a main crystal peak ($2\theta$ = 16.9, 17.9°) of S-2367 is detected on a powder X-ray diffraction chart after one week storage at 60°C, and it was revealed that S-2367 is crystallized.

[Industrial applicability]

The present preparation can improve solubility of the NPYY5 receptor antagonist in water and, moreover, can provide a preparation having high stability.

## Claims

**1.** A solid preparation comprising a compound represented by the formula (I):

[Chemical formula 1]

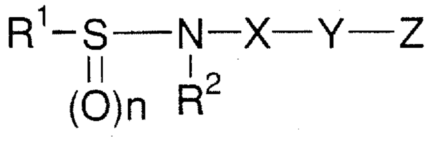

(I)

[wherein,
$R^1$ is lower alkyl optionally having a substituent, cycloalkyl optionally having a substituent, or aryl optionally having a substituent,
$R^2$ is hydrogen or lower alkyl,
$R^1$ and $R^2$ may be taken together to form lower alkylene,
n is 1 or 2,
X is lower alkylene optionally having a substituent, lower alkenylene
optionally having a substituent, -CO-lower alkylene optionally having a substituent, -CO-lower alkenylene optionally having a substituent, or

[Chemical formula 2]

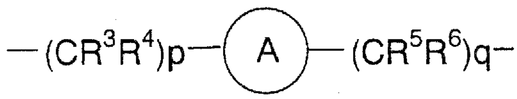

(wherein $R^3$, $R^4$, $R^5$ and $R^6$ are each independently hydrogen or lower alkyl, wherein

[Chemical formula 3]

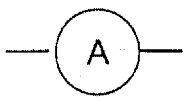

is cycloalkylene optionally having a substituent, cycloalkenylene optionally having a substituent, bicycloalkylene optionally having a substituent, arylene optionally having a substituent, or heterocyclic diyl optionally having a substituent, and p and q are each independently 0 or 1)
$-NR^2-X-$ is

[Chemical formula 4]

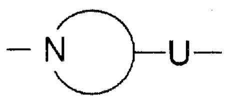

(wherein

[Chemical formula 5]

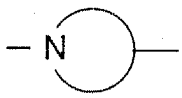

is piperidinediyl, piperazinediyl, pyridinediyl, pyrazinediyl, pyrrolidinediyl or pyrrolediyl, and U is a single bond, lower alkylene or lower alkenylene),
Y is $OCONR^7$, $CONR^7$, $CSNR^7$, $NR^7CO$ or $NR^7CS$,
$R^7$ is hydrogen or lower alkyl,
Z is lower alkyl optionally having a substituent, lower alkenyl optionally having a substituent, amino optionally having a substituent, lower alkoxy optionally having a substituent, a hydrocarbon cyclic group optionally having a substituent, or a heterocyclic group optionally having a substituent]
or a prodrug thereof, or a pharmaceutically acceptable salt thereof, or a solvate thereof, and an amorphous stabilizer.

**2.** The solid preparation according to claim 1, wherein the amorphous stabilizer is one or more selected from the group consisting of polyvinylpyrrolidone, celluloses, crosslinked polyvinylpyrrolidone, polyvinyl alcohol, polyvinyl acetate, vinyl alcohol/vinyl acetate copolymer, ethylene/vinyl acetate copolymer, polyethylene oxide derivative, sodium polystyrenesulfonate, gelatin, starch, dextran, agar, sodium alginate, pectin, pullulan, xanthan gum, acacia, chondroitin sulfate or a sodium salt thereof, hyaluronic acid, chitin, chitosan, $\alpha$, $\beta$ or $\gamma$-cyclodextrin, alginic acid derivative acryl resins, polyvinylacetal diethylaminoacetate, silicon dioxide, carrageenan and aluminum hydroxide.

**3.** The solid preparation according to claim 1 or 2, wherein the amorphous stabilizer is one or more selected from the group consisting of polyvinylpyrrolidone, celluloses, polyvinyl alcohol, polyvinyl acetate, gelatin, agar, sodium alginate, pectin, pullulan, xanthan gum, acacia, chondroitin sulfate, hyaluronic acid and carrageenan.

**4.** The solid preparation according to any one of claims 1 to 3, wherein the amorphous stabilizer is one or more celluloses selected from the group consisting of hydroxypropylcellulose, hydroxypropylmethylcellulose, hydroxypropylmethylcellulose phthalate, hydroxypropylmethylcellulose acetate succinate and carboxymethylcellulose sodium.

**5.** The solid preparation according to claim 4, wherein the amorphous stabilizer is hydroxypropylmethylcellulose phthalate and/or hydroxypropylmethylcellulose acetate succinate.

**6.** The solid preparation according to any one of claims 1 to 5, which further contains an amorphousization inducing agent.

**7.** The solid preparation according to claim 6, wherein the amorphousization inducing agent is one or more selected from the group consisting of amino acid or a salt thereof, aspartame, erythorbic acid or a salt thereof, ascorbic acid or a salt thereof, stearic acid ester, aminoethylsulfonic acid, inositol, ethylurea, citric acid or a salt thereof, glycyrrhizic acid or a salt thereof, gluconic acid or a salt thereof, creatinine, salicylic acid or a salt thereof, tartaric acid or a salt thereof, succinic acid or a salt thereof, calcium acetate, saccharine sodium, aluminum hydroxide, sorbic acid or a salt thereof, dehydroacetic acid or a salt thereof, sodium thiomalate, nicotinic acid amide, urea, fumaric acid or a salt thereof, macrogols, maltose, maltol, mannitol, meglumine, sodium desoxycholate and phosphatidylcholine.

**8.** The solid preparation according to claim 6 or 7, wherein the amorphousization inducing agent is urea and/or saccharine sodium.

**9.** The solid preparation according to any one of claims 6 to 8, wherein a content of the amorphousization inducing agent in the preparation is less than 8% by weight.

**10.** The solid preparation according to claim 9, wherein a content of the amorphousization inducing agent in the preparation is 0.1 to 6% by weight.

**11.** The solid preparation according to claim 10, wherein a content of the amorphousization inducing agent in the preparation is 2 to 4% by weight.

**12.** The solid preparation according to any one of claims 1 to 11, wherein a content of the compound represented by the formula (I), a prodrug thereof, a pharmaceutically acceptable salt thereof or a solvate thereof in the preparation is 5 to 45% by weight.

**13.** The solid preparation according to claim 12, wherein a content of the compound represented by the formula (I), a prodrug thereof, a pharmaceutically acceptable salt thereof or a solvate thereof in the preparation is 10 to 30% by weight.

**14.** The solid preparation according to any one of claims 1 to 13, wherein the compound represented by the formula (I) in the preparation is trans-N-(5-trifluoromethylpyridin-2-yl)-4-(tertiarybutylsulfonylamino)cycloh exanecarboxamide.

**15.** The solid preparation according to claim 14, which contains 5 to 45% by weight of trans-N-(5-trifluoromethylpyridin-2-yl)-4-(tertiarybutylsulfonylamino)cycloh exanecarboxamide, 40% by weight or more of hydroxypropylmethylcellulose phthalate and/or hydroxypropylmethylcellulose acetate succinate.

**16.** The solid preparation according to claim 14, which contains 10 to 30% by weight of trans-N-(5-trifluoromethylpyridin-2-yl)-4-(tertiarybutylsulfonylamino)cycloh exanecarboxamide, 70 to 90% by weight or more of hydroxypropylmethylcellulose phthalate and/or hydroxypropylmethylcellulose acetate succinate.

**17.** The solid preparation according to claim 14, which contains 10 to 20% by weight of trans-N-(5-trifluoromethylpyridin-2-yl)-4-(tertiarybutylsulfonylamino)cycloh exanecarboxamide, 80 to 90% by weight or more of hydroxypropylmethylcellulose phthalate and/or hydroxypropylmethylcellulose acetate succinate.

**18.** The solid preparation according to claim 14, which contains 5 to 45% by weight of trans-N-(5-trifluoromethylpyridin-2-yl)-4-(tertiarybutylsulfonylamino)cycloh exanecarboxamide, 40% by weight or more of hydroxypropylmethylcellulose phthalate and/or hydroxypropylmethylcellulose acetate succinate, and less than 8% by weight of urea and/or saccharin sodium.

**19.** The solid preparation according to claim 14, which contains 10 to 30% by weight of trans-N-(5-trifluoromethylpyridin-2-yl)-4-(tertiarybutylsulfonylamino) cyclohexanecarboxamide, 66 to 88% by weight or more of hydroxypropylmethylcellulose phthalate and/or hydroxypropylmethylcellulose acetate succinate, and 2 to 4% by weight of urea and/or saccharin sodium.

**20.** The solid preparation according to claim 14, which contains 10 to 15% by weight of trans-N-(5-trifluoromethylpyridin-2-yl)-4-(tertiarybutylsulfonylamino)cycloh exanecarboxamide, 81 to 88 % by weight or more of hydroxypropylmethylcellulose phthalate and/or hydroxypropylmethylcellulose acetate succinate, and 2 to 4% by weight of urea and/or saccharin sodium.

**21.** The solid preparation according to any one of claims 1 to 5, and 15 to 17, which does not contain the amorphousization inducing agent.

**22.** The solid preparation according to any one of claims 1 to 21, which is a solid dispersion.

**23.** A process for producing the solid preparation as defined in any one of claims 1 to 22, comprising using a spray drying method.

**24.** A method of improving solubility of a NPYY5 receptor antagonist, comprising adding an amorphous stabilizer to a solid preparation containing the NPYY5 receptor antagonist.

**25.** A method of improving solubility of a NPYY5 receptor antagonist, comprising adding an amorphousization inducing agent to a solid preparation containing the NPYY5 receptor antagonist and an amorphous stabilizer.

[Fig.1]

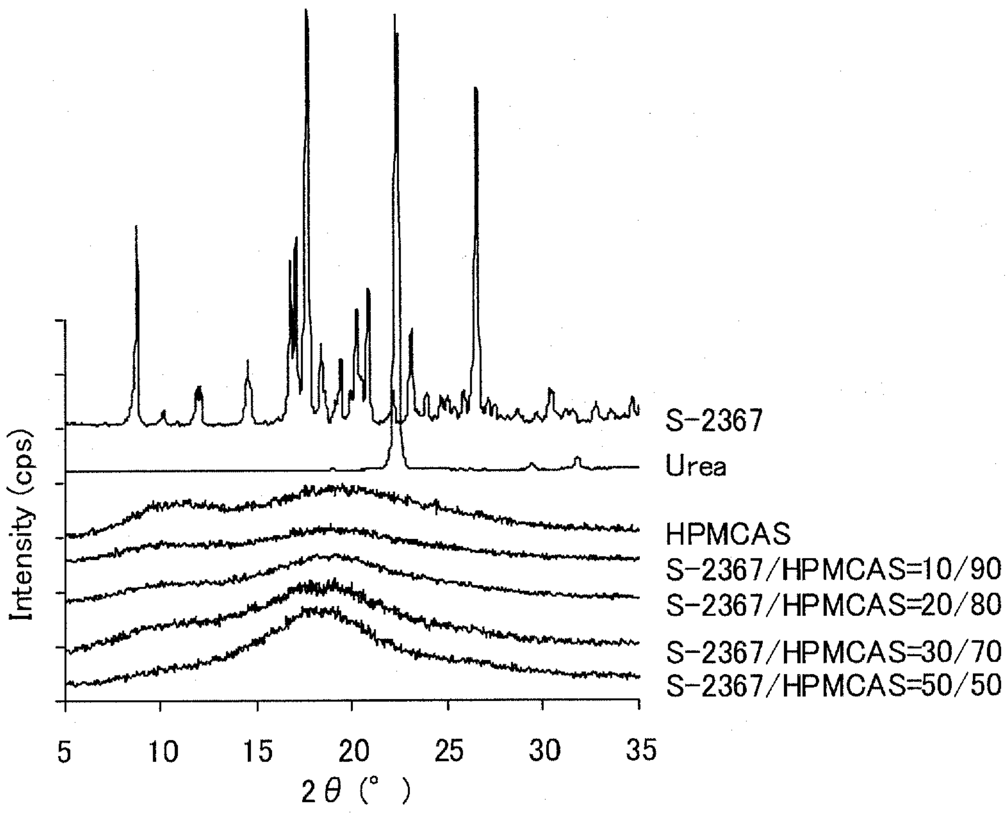
Intensity (cps)
S-2367
Urea
HPMCAS
S-2367/HPMCAS=10/90
S-2367/HPMCAS=20/80
S-2367/HPMCAS=30/70
S-2367/HPMCAS=50/50
5
10
15
20
25
30
35
2θ (° )

[Fig.2]

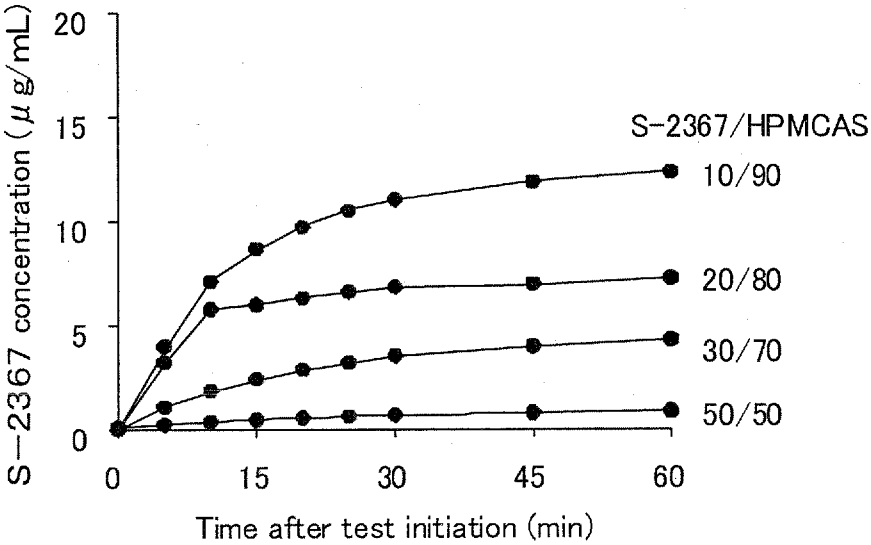
S−2367 concentration (μg/mL)
20
15
10
5
0
S-2367/HPMCAS
10/90
20/80
30/70
50/50
0
15
30
45
60
Time after test initiation (min)

[Fig.3]

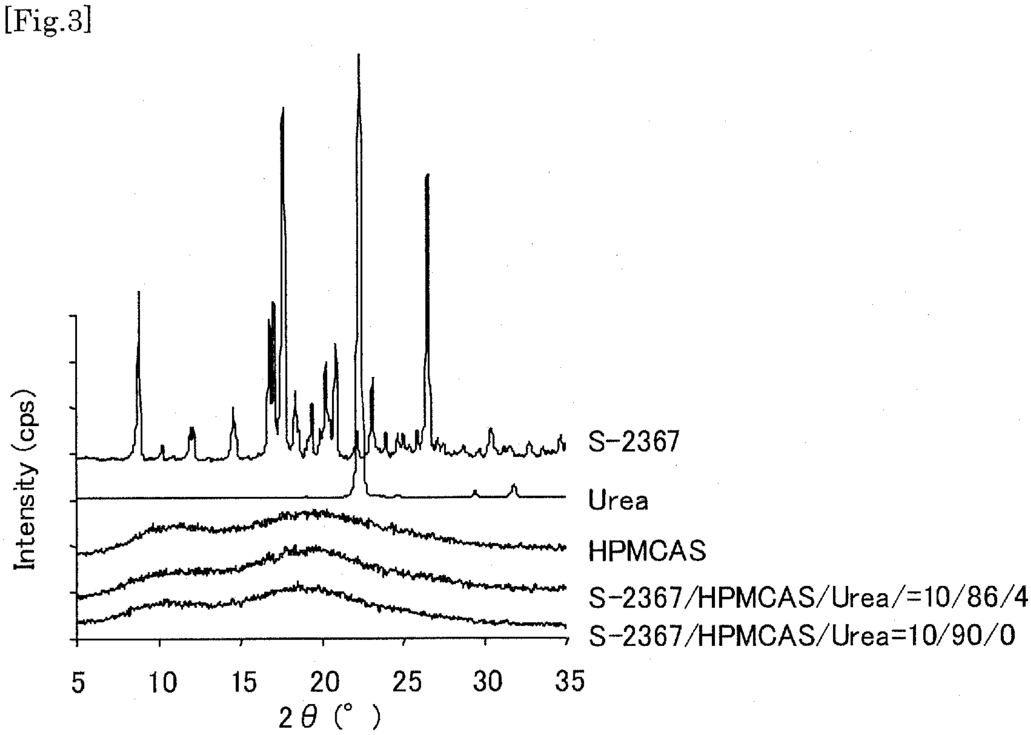
Intensity (cps)
S-2367
Urea
HPMCAS
S-2367/HPMCAS/Urea/=10/86/4
S-2367/HPMCAS/Urea=10/90/0
5
10
15
20
25
30
35
2θ (° )

[Fig.4]

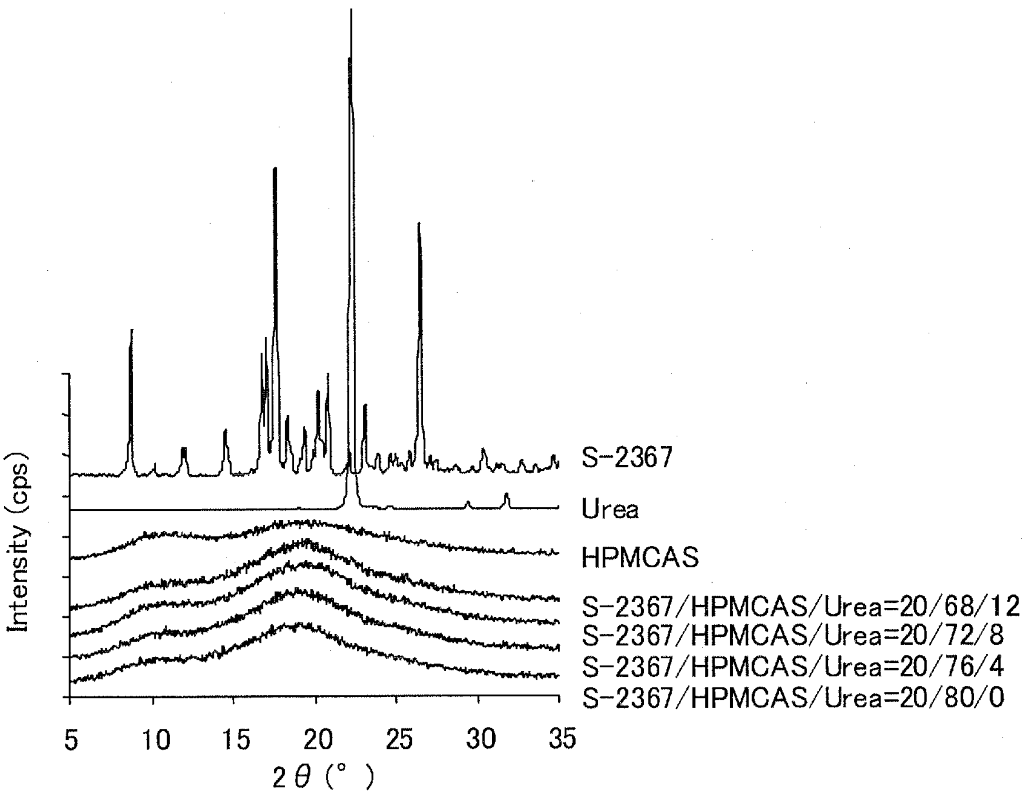
Intensity (cps)
S-2367
Urea
HPMCAS
S-2367/HPMCAS/Urea=20/68/12
S-2367/HPMCAS/Urea=20/72/8
S-2367/HPMCAS/Urea=20/76/4
S-2367/HPMCAS/Urea=20/80/0
5
10
15
20
25
30
35
2θ (° )

[Fig.5]

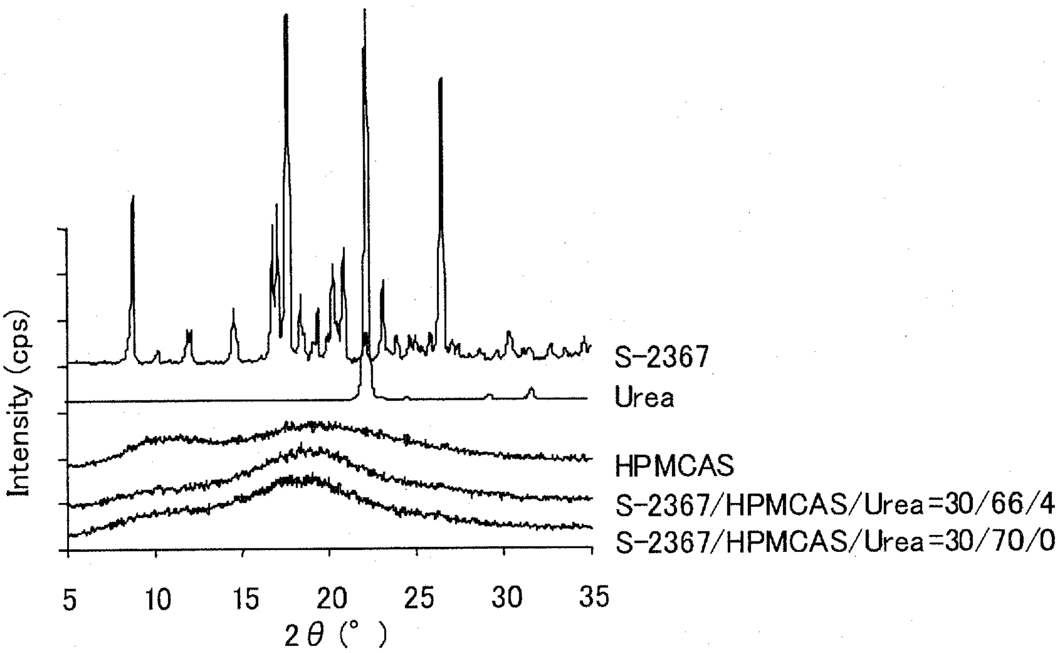
Intensity (cps)
S-2367
Urea
HPMCAS
S-2367/HPMCAS/Urea=30/66/4
S-2367/HPMCAS/Urea=30/70/0
5
10
15
20
25
30
35
2θ (° )

[Fig.6]

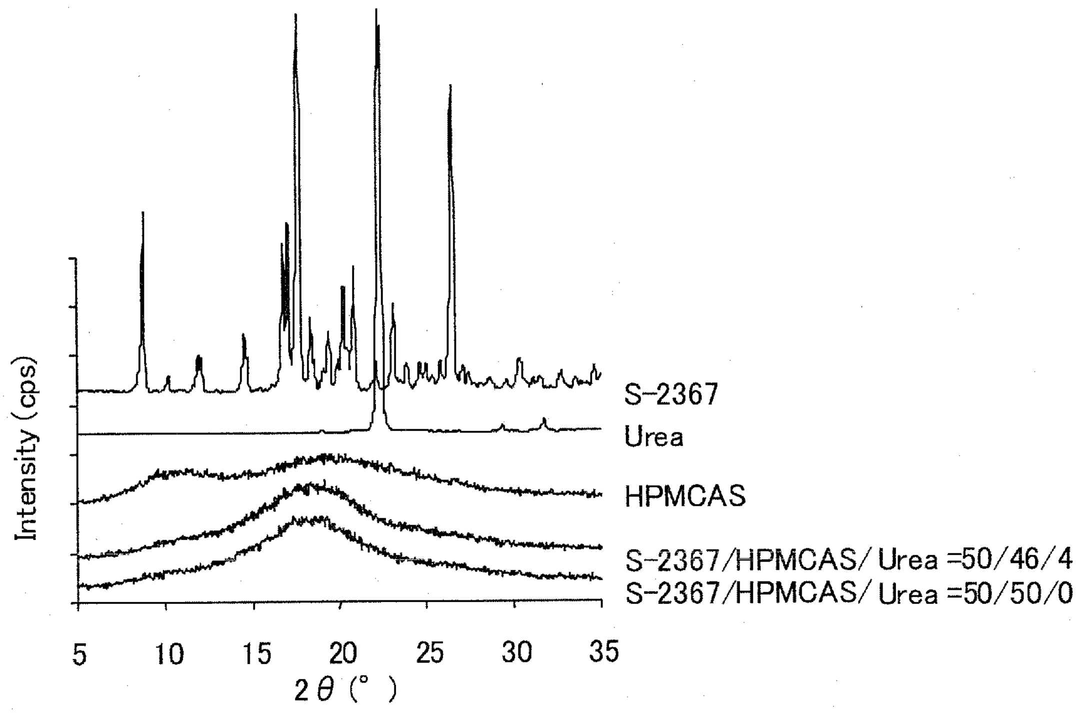
Intensity (cps)
S-2367
Urea
HPMCAS
S-2367/HPMCAS/ Urea =50/46/4
S-2367/HPMCAS/ Urea =50/50/0
5
10
15
20
25
30
35
2θ (° )

[Fig.7]

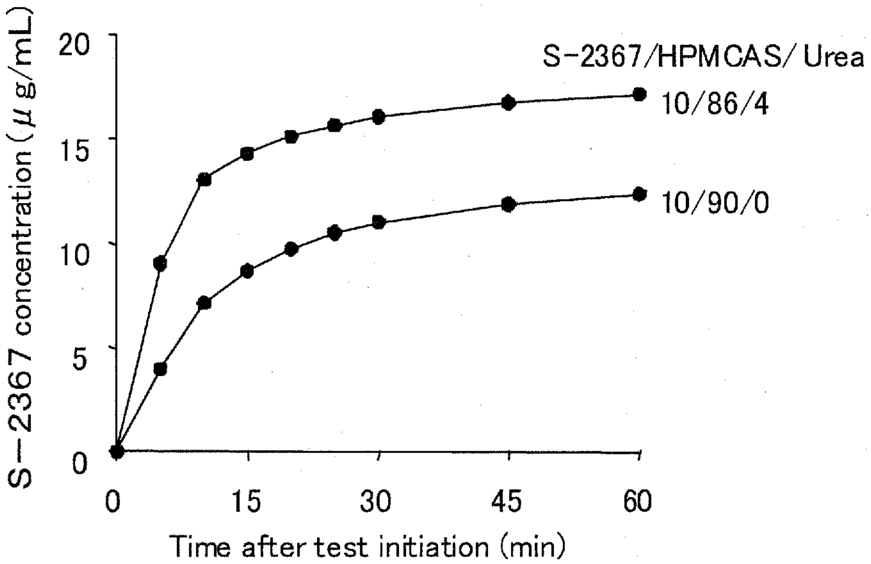
S−2367 concentration (μg/mL)
20
15
10
5
0
0
15
30
45
60
Time after test initiation (min)
S-2367/HPMCAS/ Urea
10/86/4
10/90/0

[Fig.8]

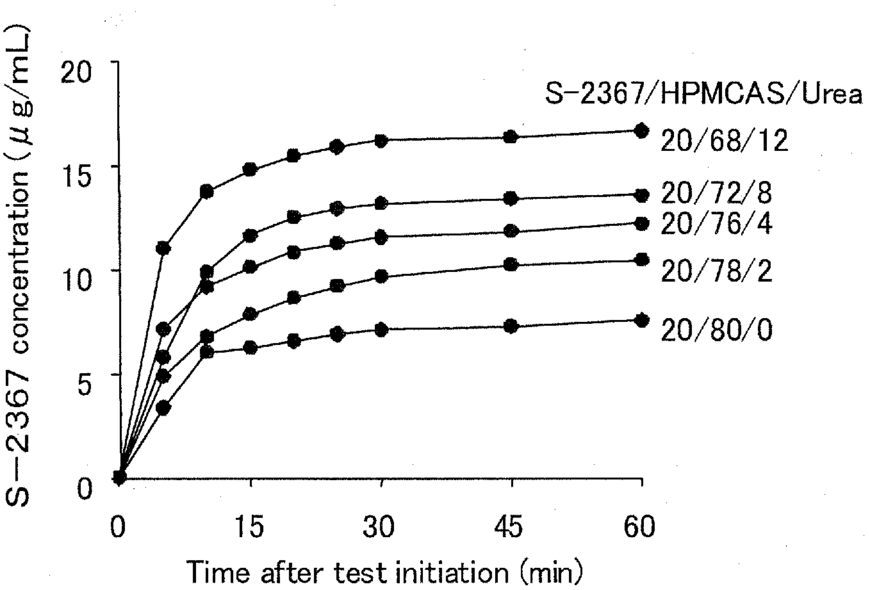
S−2367 concentration (μg/mL)
20
15
10
5
0
0
15
30
45
60
Time after test initiation (min)
S-2367/HPMCAS/Urea
20/68/12
20/72/8
20/76/4
20/78/2
20/80/0

[Fig.9]

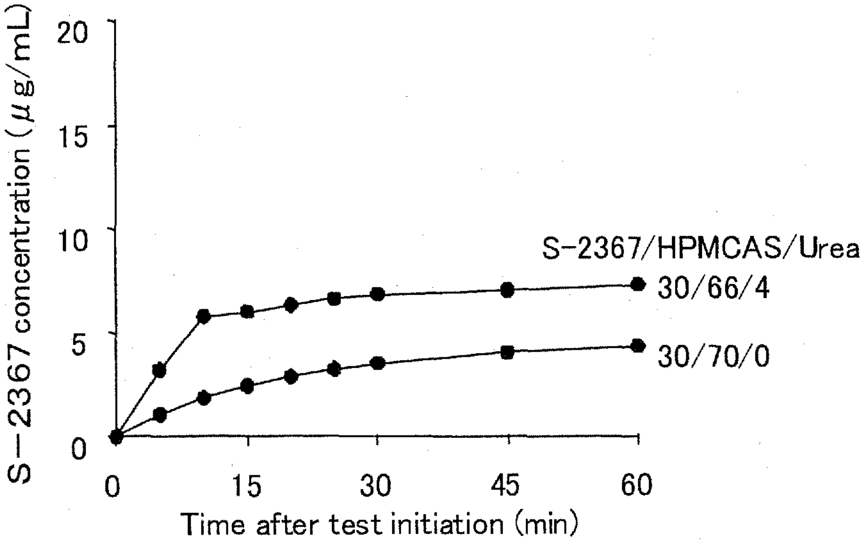
S−2367 concentration (μg/mL)
20
15
10
5
0
0
15
30
45
60
Time after test initiation (min)
S-2367/HPMCAS/Urea
30/66/4
30/70/0

[Fig.10]

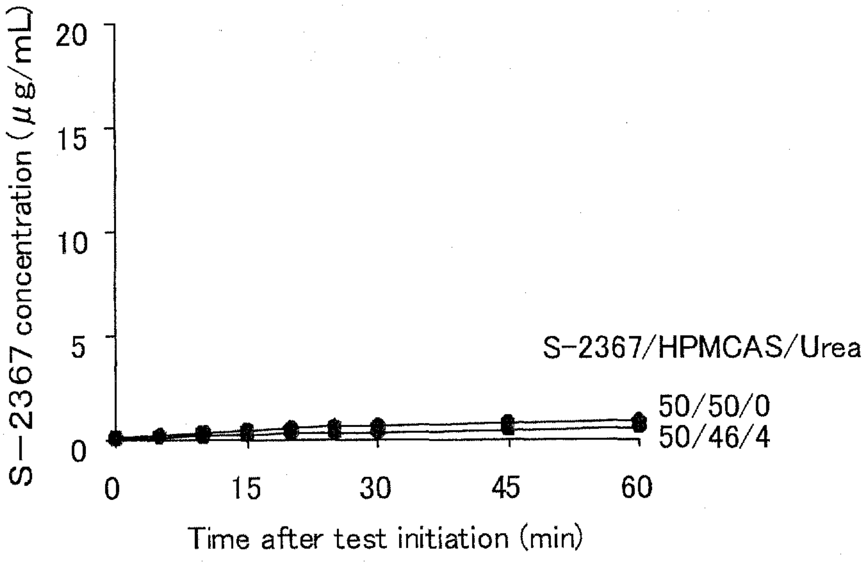
S−2367 concentration (μg/mL)
20
15
10
5
0
0
15
30
45
60
Time after test initiation (min)
S-2367/HPMCAS/Urea
50/50/0
50/46/4

[Fig.11]

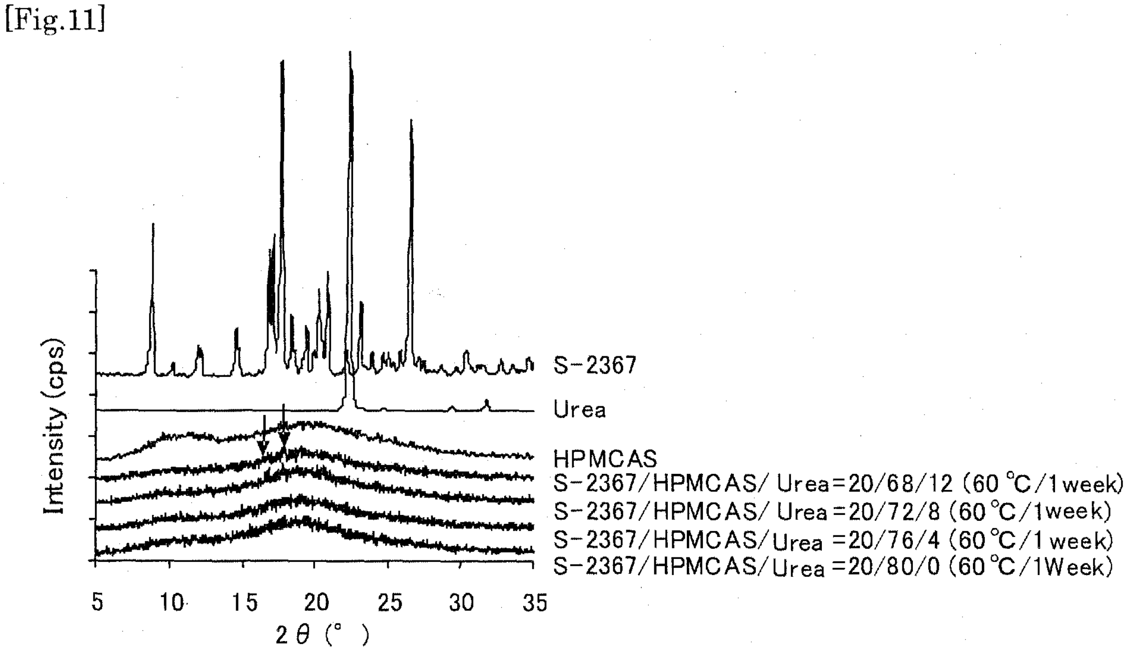
Intensity (cps)
S-2367
Urea
HPMCAS
S-2367/HPMCAS/ Urea=20/68/12 (60 °C/1week)
S-2367/HPMCAS/ Urea=20/72/8 (60 °C/1week)
S-2367/HPMCAS/Urea =20/76/4 (60 °C/1 week)
S-2367/HPMCAS/Urea =20/80/0 (60 °C/1Week)
5 10 15 20 25 30 35
2θ (° )

[Fig.12]

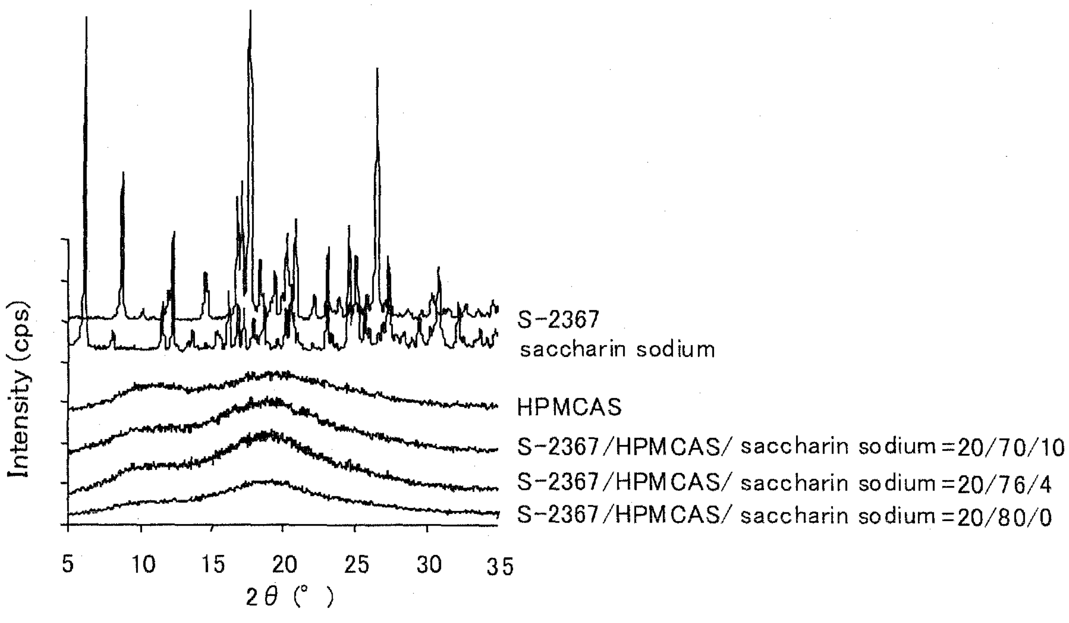
Intensity (cps)
S-2367
saccharin sodium
HPMCAS
S-2367/HPMCAS/ saccharin sodium=20/70/10
S-2367/HPMCAS/ saccharin sodium=20/76/4
S-2367/HPMCAS/ saccharin sodium=20/80/0
5 10 15 20 25 30 35
2θ (° )

[Fig.13]

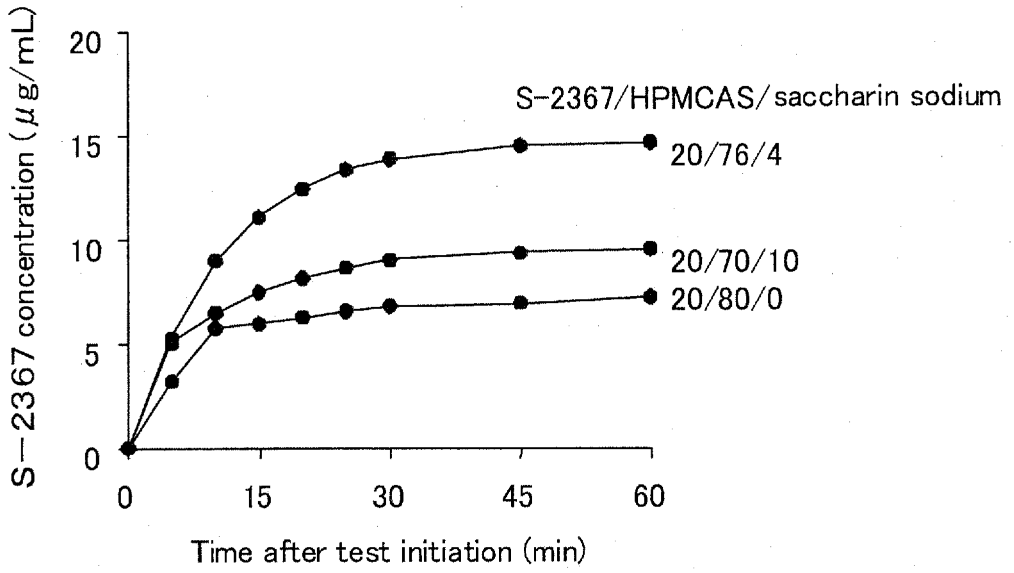
S−2367 concentration (μg/mL)
20
15
10
5
0
0
15
30
45
60
Time after test initiation (min)
S-2367/HPMCAS/saccharin sodium
20/76/4
20/70/10
20/80/0

[Fig.14]

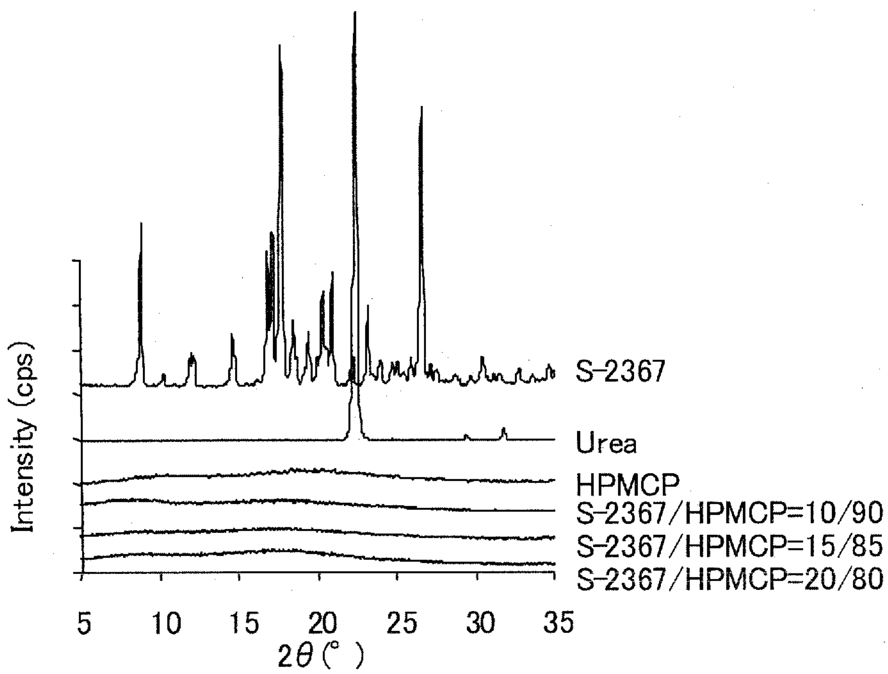
Intensity (cps)
S-2367
Urea
HPMCP
S-2367/HPMCP=10/90
S-2367/HPMCP=15/85
S-2367/HPMCP=20/80
5
10
15
20
25
30
35
2θ(° )

[Fig.15]

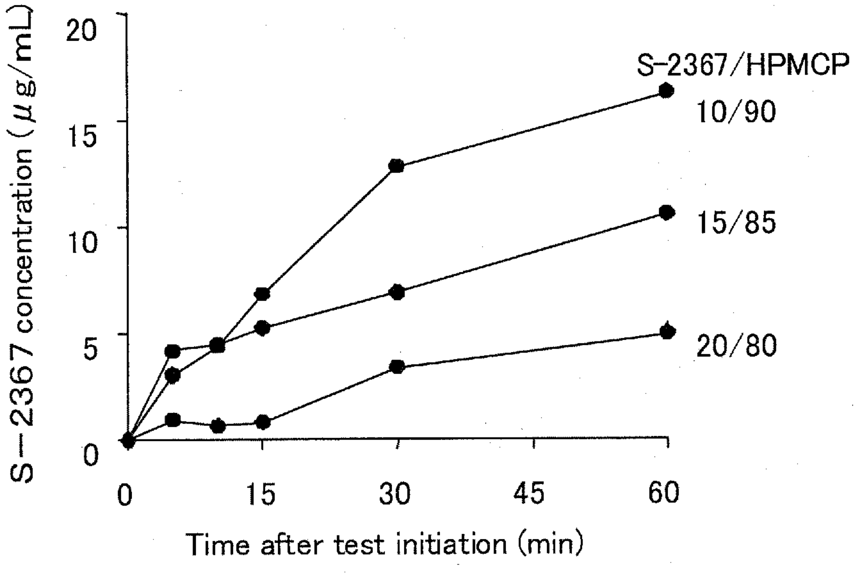
S−2367 concentration (μg/mL)
20
15
10
5
0
0
15
30
45
60
Time after test initiation (min)
S-2367/HPMCP
10/90
15/85
20/80

[Fig.16]

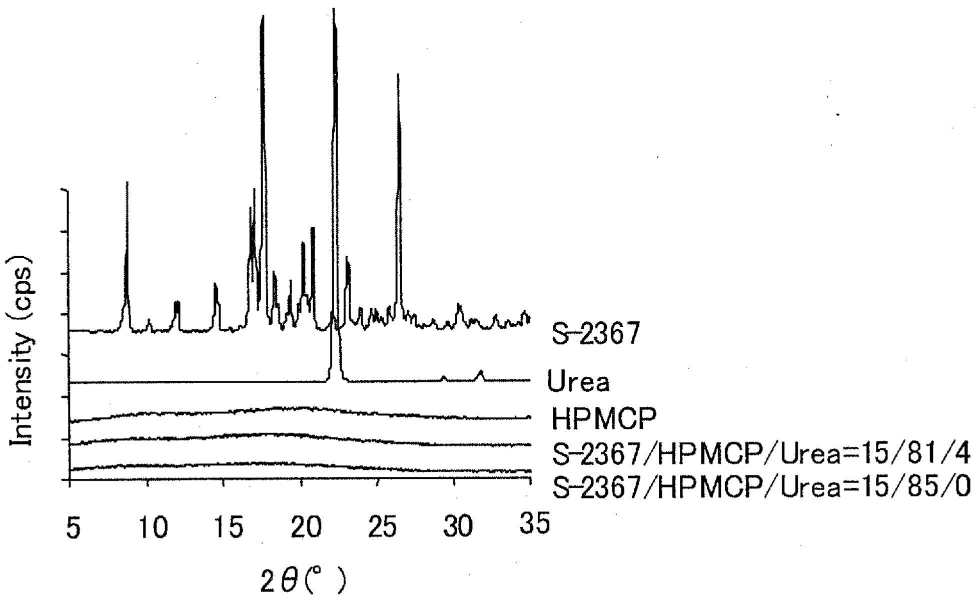
Intensity (cps)
S-2367
Urea
HPMCP
S-2367/HPMCP/Urea=15/81/4
S-2367/HPMCP/Urea=15/85/0
5
10
15
20
25
30
35
2θ(° )

[Fig.17]

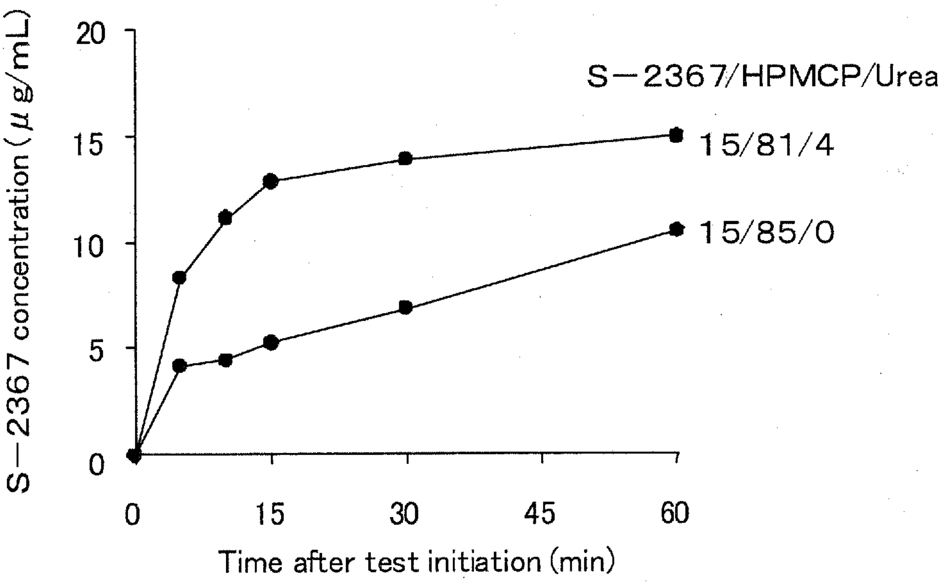
S−2367 concentration (μg/mL)
20
15
10
5
0
S−2367/HPMCP/Urea
15/81/4
15/85/0
0
15
30
45
60
Time after test initiation (min)

[Fig.18]

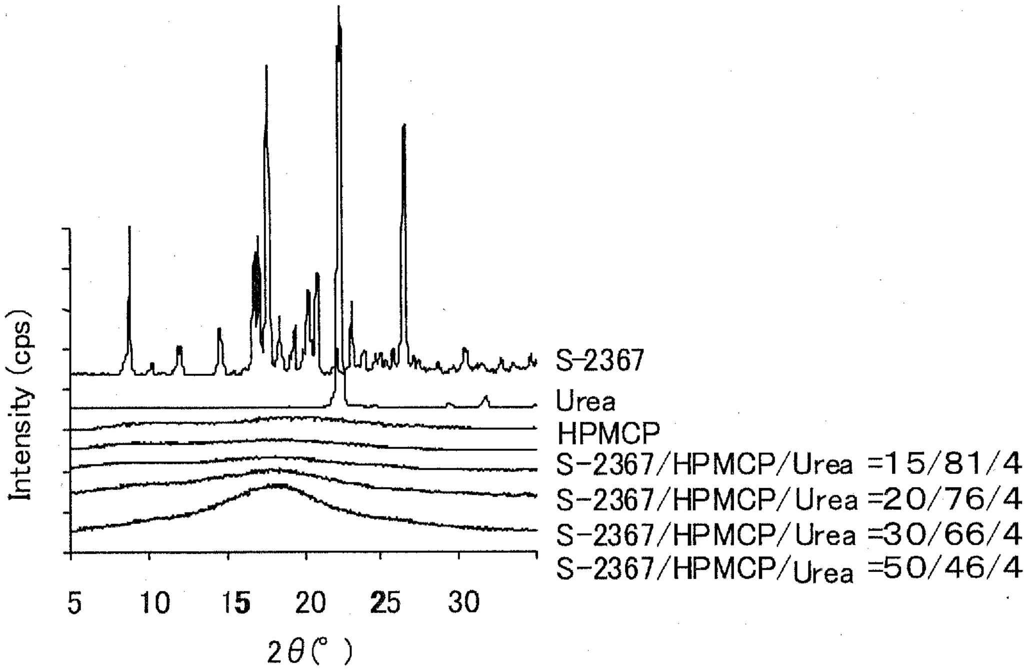
Intensity (cps)
S-2367
Urea
HPMCP
S-2367/HPMCP/Urea =15/81/4
S-2367/HPMCP/Urea =20/76/4
S-2367/HPMCP/Urea =30/66/4
S-2367/HPMCP/Urea =50/46/4
5
10
15
20
25
30
2θ(°)

[Fig.19]

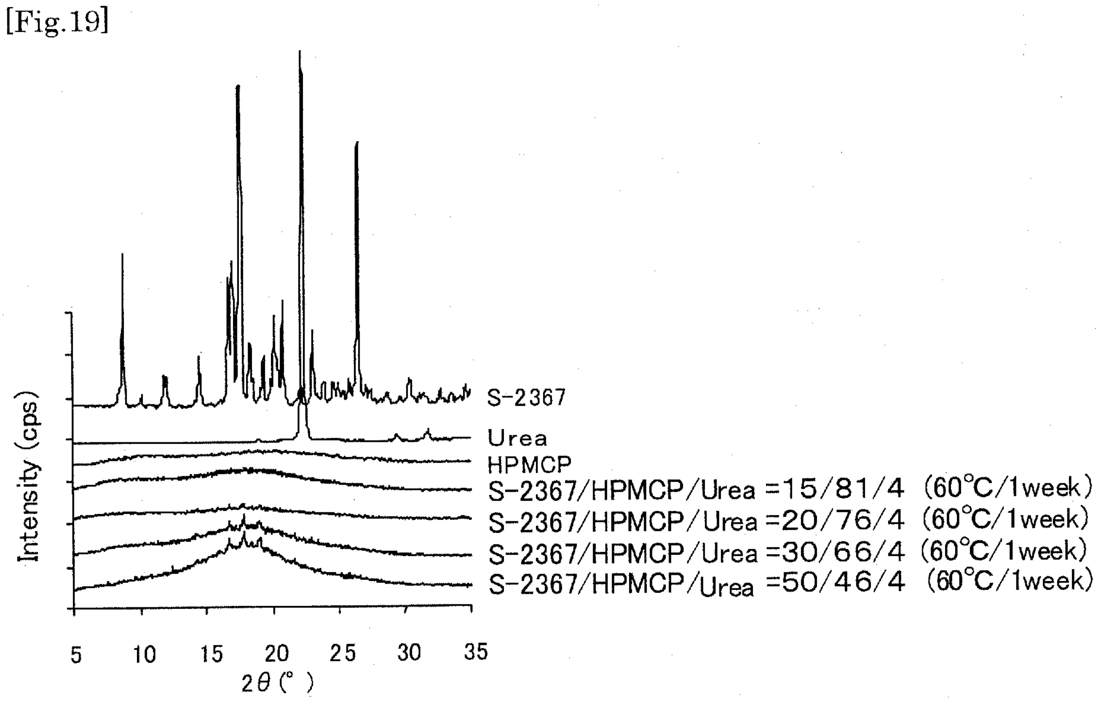
Intensity (cps)
S-2367
Urea
HPMCP
S-2367/HPMCP/Urea =15/81/4 (60°C/1week)
S-2367/HPMCP/Urea =20/76/4 (60°C/1week)
S-2367/HPMCP/Urea =30/66/4 (60°C/1week)
S-2367/HPMCP/Urea =50/46/4 (60°C/1week)
5 10 15 20 25 30 35
2θ(°)

**INTERNATIONAL SEARCH REPORT**

International application No.
PCT/JP2008/066818

A. CLASSIFICATION OF SUBJECT MATTER
*A61K31/44*(2006.01)i, *A61K47/16*(2006.01)i, *A61K47/22*(2006.01)i, *A61K47/38*(2006.01)i, *A61P25/02*(2006.01)i, *A61P25/16*(2006.01)i, *A61P25/22*(2006.01)i, *A61P25/24*(2006.01)i, *A61P25/28*(2006.01)i, *A61P43/00*(2006.01)i,
According to International Patent Classification (IPC) or to both national classification and IPC

B. FIELDS SEARCHED
Minimum documentation searched (classification system followed by classification symbols)
A61K31/44, A61K47/16, A61K47/22, A61K47/38, A61P25/02, A61P25/16, A61P25/22, A61P25/24, A61P25/28, A61P43/00, C07D213/76

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
CA(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | WO 01/37826 A1 (Shionogi & Co., Ltd.), 31 May, 2001 (31.05.01), Full text & JP 3910446 B & US 2004/0180964 A1 & US 6699891 B1 & US 2004/0176462 A1 & EP 1249233 A1 | 1-25 |
| Y | WO 03/76374 A1 (Shionogi & Co., Ltd.), 18 September, 2003 (18.09.03), Full text & JP 3850838 B & US 2005/0222255 A1 & EP 1484301 A1 | 1-25 |
| Y | WO 06/64906 A1 (Ono Pharmaceutical Co., Ltd.), 22 June, 2006 (22.06.06), Full text & US 2008/0096924 A & EP 1829549 A1 | 1-25 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>28 October, 2008 (28.10.08) | Date of mailing of the international search report<br>04 November, 2008 (04.11.08) |
| Name and mailing address of the ISA/<br>Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2008/066818

C (Continuation). DOCUMENTS CONSIDERED TO BE RELEVANT

| Category* | Citation of document, with indication, where appropriate, of the relevant passages | Relevant to claim No. |
|---|---|---|
| Y | WO 92/18106 A1 (Nippon Shinyaku Co., Ltd.), 29 October, 1992 (29.10.92), Full text & JP 2527107 B & US 5456923 A & US 5811547 A & EP 580860 A1 | 1-25 |
| Y | JP 11-246417 A (Nissan Chemical Industries, Ltd.), 14 September, 1999 (14.09.99), Full text (Family: none) | 1-25 |
| Y | EP 901786 A2 (Pfizer Products Inc.), 17 March, 1999 (17.03.99), Full text & JP 11-116502 A & US 2002/0009494 A1 & US 2003/0219489 A1 & EP 1741424 A2 & EP 1745774 A2 | 1-25 |
| Y | WO 97/06781 A1 (Nissan Chemical Industries, Ltd.), 27 February, 1997 (27.02.97), Full text & US 6462093 B1 & EP 852140 A1 & WO 1997/006781 A1 | 1-25 |

Form PCT/ISA/210 (continuation of second sheet) (April 2007)

**INTERNATIONAL SEARCH REPORT**

International application No.
PCT/JP2008/066818

Continuation of A. CLASSIFICATION OF SUBJECT MATTER
(International Patent Classification (IPC))

*C07D213/76*(2006.01)i

(According to International Patent Classification (IPC) or to both national classification and IPC)

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0137826 A
- WO 9706781 A
- JP 9309834 A
- JP 2049728 A
- JP 2004528358 A
- JP 2004143185 A
- JP 2002529519 A
- WO 2007108463 A
- WO 03076374 A


### Non-patent literature cited in the description


- *Trends in Pharmacological Sciences,* 1994, vol. 15, 153
- *Trends in Pharmacological Sciences,* 1997, vol. 18, 372
- *Peptides,* 1997, vol. 18, 445
- *2005 Report of Important Study of Drug Design etc. Human Science Study,* 31 July 2006
- Design of Prodrugs. Elsevier, 1985